# EUROPEAN PATENT SPECIFICATION

(11) **EP 1 287 016 B1**
(45) Date of publication and mention of the grant of the patent: **15.09.2004**
(21) Application number: 01944795.2
(22) Date of filing: 08.06.2001
(51) Int. Cl.: C07J 9/00, C07J 41/00, A61K 49/04, A61K 51/04

(54) **RADIOIMAGING PROBES AND THE USE THEREOF**
RÖNTGENDIAGNOSTIKA
SONDES DE RADIOIMAGERIE ET UTILISATION DE CES DERNIERES

(30) Priority: 08.06.2000 CA 2311209
(43) Date of publication of application: 05.03.2003
(73) Proprietor: Genesis Group Inc., St. John's, Newfoundland, A1B 3X5 (CA)
(72) Inventor: LIU, Hu, Newfoundland A1B 4M2 (CA); WANG, Lili, Newfoundland A1B 4M2 (CA); XIAO, Wu, Gainesville, FL 32603 (US)
(74) Representative: Scott, Susan Margaret
(86) International application number: PCT/CA2001/000822
(87) International publication number: WO 2001/093918

(56) References cited:
- WO-A-96/28414
- US-A- 4 933 157
- US-A- 5 725 840
- W. XIAO ET AL.: "Radiolabeled cholesteryl Iopanoate/Acetylated low Density Lipoprotein as aPotential Probe for Visualization of Early atherosclerotic Lesions in Rabbits." PHARMACEUTICAL RESEARCH, vol. 16, no. 3, 1999, pages 420-426, XP002942252 NEW YORK, NY, US ISSN: 0724-8741 cited in the application
- XIAO WU ET AL.: "Microemulsion of seal oil Markedly Enhances the Transfer of a hydrophobic Radiopharmaceutical into acetylated low Density Lipoprotein" LIPIDS, vol. 34, no. 5, 1999, pages 503-509, XP001057044 cited in the application
- JAMEY P. WEICHERT ET AL.: "Polyiodinated Triglyceride Analogs as Potential Computed Tomography Imaging Agents for the Liver." JOURNAL OF MEDICINAL CHEMISTRY., vol. 38, no. 4, 1995, pages 636-646, XP001057012 AMERICAN CHEMICAL SOCIETY., US ISSN: 0022-2623 cited in the application
- DOUGLAS A. BAKAN ET AL.: "Physicochemical Characterization of a Synthetic lipid Emulsion for Hepatocyte-Selective Delivery of Lipophilic Compounds: Application to Polyiodinated Triglycerides as Contrast Agents for Computed Tompgraphy" JOURNAL OF PHARMACEUTICAL SCIENCES, vol. 85, no. 9, 1996, pages 908-914, XP001056890 AMERICAN CHEMICAL SOCIETY, AMERICAN PHARMACEUTICAL ASSOCIAION cited in the application
- W. XIAO ET AL.: "Incorporation of an 125I-Labeled Hexa-Iodinated Diglyceride Analog Into ow-Density Lipoprotein and high Specific Uptake by Cells of Cervical Carcinoma Cell Lines." RADIATION RESEARCH, vol. 152, 1999, pages 250-256, XP001057056 ACADEMIC PRESS INC., US ISSN: 0033-7587

## Description

### FIELD OF THE INVENTION

This invention pertains to the field of radioimaging probes, and more specifically to radioimaging probes that are polyiodinated diacylglyceryl (IDG) ester and ether analogues as and their use as diagnostic radiolabeled probes for clinical radioimaging.

### BACKGROUND

Radioimaging probes are valuable tools in the diagnosis of a number of clinically relevant diseases. Nuclear medicine techniques, such as computer tomography (CT) and radionucleotide scintigraphy, employ these probes as a non-invasive means of providing important functional and biochemical information about a particular organ or area of interest. The success of nuclear imaging techniques depends largely upon the selective uptake of the probe molecule by the target tissue, which in turn depends on the development of probes with a high degree of specificity for the target tissue.

Probe molecules for radioimaging have been developed that possess certain inherent physical characteristics allowing them to target specific cell types. For example, in order to take advantage of the high concentration of phospholipid ethers in the cell membranes of tumor cells relative to normal cells, radioiodinated medium- and long-chain phospholipid ethers have been developed. Administration of these ethers solubilized in Tween-20 to rats that were subsequently inoculated with a carcinosarcoma cell line resulted in selective accumulation of the radiolabel in the tumor cells (Canadian Patent Application No. 2,276,284).

An alternative method of targeting radioimaging probes to specific areas or tissues is to incorporate them in a targeted delivery vehicle. Examples of such vehicles include liposomes, chemically modified low-density lipoprotein (LDL), and synthetic emulsions. The utility of such lipid-based delivery vehicles lies in the fact that they mimic natural compounds found in the body. Chemically modified LDL in particular has considerable potential as a targeted delivery vehicle due to the presence of specific receptors for both native LDL and modified LDL in a variety of human tissues including hepatocytes, peripheral blood lymphocytes, smooth muscle cells, macrophages and neurons of the central nervous system (CNS). Receptors for modified LDL, the AcLDL or "scavenger" receptors, are responsible for the binding, internalization and subsequent clearance of lipoproteins modified by oxidation, glycation, alkylation or nitration. As such, scavenger receptors are found mainly on activated macophages, dendritic cells and the Kupffer cells of the liver. However, scavenger receptor expression also occurs in other cells including endothelial cells, aortic smooth muscle cells, neuronal cells and keratinocytes. Diseases accompanied by oxidation of lipoprotein, such as atherosclerosis, Alzheimer disease, glomerulosclerosis and ataxia with Vitamin E deficiency may, therefore, share the common feature of unregulated expression of scavenger receptors [Zingg *et al., IUBMB Life,* **49**:397-443 (2000)]. Deregulation of LDL receptor expression and / or increased LDL receptor activity has also been shown to be a feature of a number of cancer cell lines [Ho, *et al., Blood,* **52**:1099-1104 (1978); Rudling, *et al., Cancer Res.,* **50**:483-487 (1990); Chen and Hughes-Fulford, *Int. J. Cancer,* **91**:41-45 (2001)], and the potential of LDL as a carrier for anticancer drugs has been investigated [Gal, *et al., Am. J. Obstet. Gynecol,.* **139**:877-885 (1981); Masquelier, *et al., Cancer Res,.* **46**:3842-3947 (1986); Lundberg, *Cancer Res.,.* **47**:4105-4108 (1987); Firestone, *Bioconjug. Chem.,* **5**:105-113 (1994)].

Two major limitations, however, are associated with the use of LDL as a delivery vehicle. Firstly, the modified LDL has to compete for receptors in the target tissue with a large population of native LDL. This competition necessitates high levels of probe incorporation into the carrier to ensure that sufficient amounts are delivered to the target cells. Secondly, radioimaging probes developed for delivery by LDL particles usually consist of either surface components (*i.e* apolipoprotein B-100 (apo B), or short peptides based on the apo B sequence), or core components (*i.e.* cholesteryl ether (CE)) labeled with an appropriate radioisotope. Both apo B and CE are extremely susceptible to hydrolysis by lysosomal enzymes *in vivo*. Subsequent transport of the hydrolysed fragments out of the target cells leads to a dissipation of the radiosignal and poor image quality. In addition, since apo B is critical for the interaction of LDL with its cellular receptor, radiolabeling this surface element may interfere with the native LDL-receptor interaction. Probes based on the apo B protein, however, have met with some success. For example, a radiolabeled short peptide based on the sequence of apo B is currently showing promising results in a multi-center U.S. clinical trial [Lees and Lees, *Atherosclerosis X,* pp.999-1000, Elsevier Scientific (1995)].

In order to circumvent interference of the LDL to receptor binding, core components of the LDL have been labeled. A further potential advantage of labeling core components is that certain cells, notably those of the liver, adrenals and ovary, are able to take up a disproportionately greater amount of core lipid than surface protein [Glass *et al., Proc. Natl. Acad. Sci. USA,* **80**: 5435-5439 (1983); Glass *et al.,J. Biol. Chem.,* **260**: 744-750 (1985); Leitersdorf *et al., Biochim. Biophys. Acta,* **878**: 320-329 (1986)], which may help to increase the specificity of CE-based probes. In view of the ready hydrolysis of native CE, however, efforts to develop probes based on core LDL components have been directed to analogues that show increased resistance to hydrolysis.

One non-hydrolysable analogue of CE that has been developed is cholesteryl iopanoate (CI) [Seevers *et al., J. Med. Chem,* **25**: 1500-1503 (1982)]. Evaluation of radiolabeled CI as a means of targeting imaging probes to specific tissues has confirmed the potential of lipoproteins as delivery vehicles. Preliminary studies demonstrated that ¹²⁵I-labeled CI accumulated preferentially in atheroma relative to normal arterial tissue [DeGalen *et al., Pharmaceut. Res.,* **3**: 52-55 (1986)], and more recent studies reported successful detection of atherosclerotic lesions using a radiolabeled CI-acetylated LDL (AcLDL) conjugate [Xiao, *et al., Pharm. Res.,* **16**: 420-426 (1999)]. The radiolabeled CI conjugate was shown to selectively localize to the atherosclerotic lesion areas of the arteries, demonstrating the utility both of CI as a probe molecule and of chemically modified lipoproteins, such as AcLDL, as targeted delivery vehicles.

LDL particles contain both CE and triglycerides in the inner core. In another study a hydrolysis-resistant probe was developed that was based on the triglyceride structure. This compound, 1,3-dihydroxypropan-2-one 1,3-diiopanoate (DPIP), was radiolabeled with ¹²⁵I and incorporated into AcLDL. The ¹²⁵I-DPIP / AcLDL conjugate was shown to selectively taken up by cervical cancer cell lines [Xiao *et al., Radiat. Res.,* **152**:250-256 (1999)].

Radioimaging probes dispersed in microemulsions have also been developed for targeted delivery to the liver. The specific targeting properties of microemulsions are believed to be due to their close resemblance to chylomicron remnants. Chylomicrons are naturally occurring lipoproteins that transport triglycerides and cholesterol away from the intestinal tract. Endogenous lipases hydrolyse the triglyceride component of the chylomicrons and the resulting cholesterol-rich residues are known as chylomicron remnants. Like other lipoproteins, chylomicron remnants are associated with certain apoproteins; in this case apo B and apo E. Chylomicron remnants are cleared rapidly from the circulation by the liver through a receptor-mediated process that recognizes apo B and apo E.

One of the more promising emulsion-based radioprobes, an emulsion of iodinated poppyseed oil in saline known as EOE-13, has been extensively studied in animals and humans in the U.S. Although this probe provided good targeted delivery to hepatic cells and acceptable diagnostic efficiency, a high incidence of adverse side-effects was reported [Miller *et al., Am. J. Radiol.,* **143**: 235-243 (1984)].

Other microemulsion-based radioimaging probes include iodinated triglycerides in synthetic lipid emulsions that are targeted to hepatic cells. These triglycerides, the structure of which is based on 2-oleoylglycerol-1,3-bis[ω-(3-amino-2,4,6-triiodophenyl)alkanoates], showed high levels of incorporation into the emulsions and good hepatocyte selectivity [U.S. Patent No. 5,654,452; Weichert *et al., J. Med. Chem.,* **17**:636-646 (1995); Weichert *et al., J.Pharm. Sci.,* **85**:908-914 (1996)].

Recently, a novel method of using microemulsions to enhance the incorporation of a radiopharmaceutical into LDL has been developed. This method utilizes a microemulsion as a donor particle to transfer the radioimaging probe DPIP to AcLDL particles with high efficiency [Xiao *et al.*, *Lipids,* **34**: 503-509 (1999)]. Several core lipids were tested in the donor microemulsions including triolein, canola oil, squalene and seal oil, with seal oil proving the most efficient.

LDL and microemulsions have, therefore, demonstrated good practical utility for the targeted delivery of diagnostic and therapeutic compounds. However, in order to capitalize on the potential of these delivery vehicles in the field of clinical radioimaging, the level of incorporation of the radiolabeled probe into the delivery vehicle must be maximized. Improved probe molecules that demonstrate high levels of incorporation into LDL particles and microemulsions for targeted delivery would provide a significant advancement in the field of diagnostic radioimaging.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

An object of this invention is to provide novel radioimaging probes. In accordance with one aspect of the present invention there is provided a compound of formula (I): 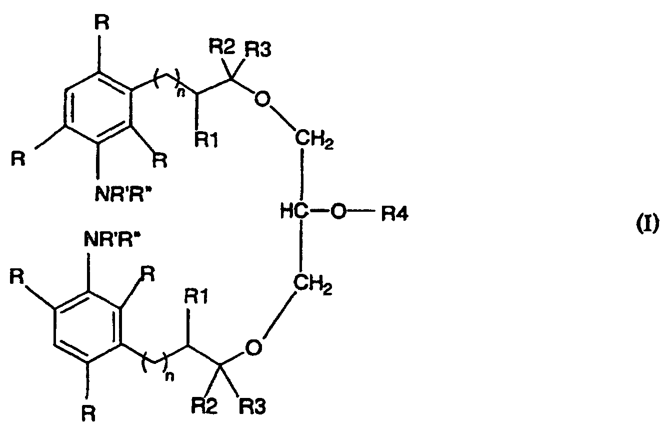 wherein:
n is an integer between 1 and 16;
each R group is independently H or I, wherein between 2 to 6 R groups must I;
R' and R" are independently H, (C1-C4 alkyl) or an amine protecting group;
R1 is H or (C₁-C₆) alkyl;
R2 is H;
R3 is H or when taken together R2 and R3 is =O; and
R4 is: 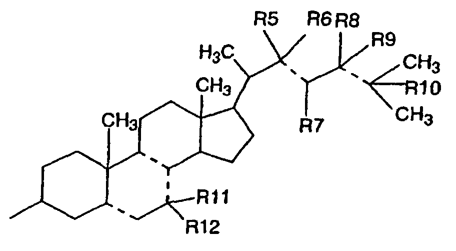 wherein:
   the bond - - - designates a single or a double bond;
   R5 is H, OR"' or R"', wherein R"' is (C₁-C₆) alkyl;
   R6 is H or absent;
   R7 is H or absent;
   R8 is H, (C₁-C₆) alkyl or (C₂-C₆) alkenyl;
   R9 is H or absent;
   R10 is H or absent;
   R11 and R12 are independently H, R"' or OR"', or when taken together R11 and R12 are =O;
   with the proviso that when R9 and R10 are absent and the bond - - - between carbon atoms bearing R9 and R10 is a double bond, then R8 is H or (C₁-C₆ alkyl; and that two adjacent bonds designated as - - - are not double bond at the same time.

In accordance with another aspect of the present invention there is provided a composition comprising one or more compounds of formula (I) and a suitable delivery vehicle. Examples of suitable delivery vehicles include, but are not limited to, liposomes, low density lipoprotein (LDL), modified LDL and synthetic microemulsions.

The present invention also provides for the use of compounds of formula (I) and compositions comprising these compounds for diagnostic radioimaging. The compounds and compositions of the present invention can be used to visualize tissues or organs in a mammal that contain high concentrations of LDL receptors, such as atherosclerotic lesions, liver cells, neural cells and tumour cells. It is envisioned that the compounds and compositions of the present invention may be used for diagnostic purposes to determine the presence and / or extent of a disease that affects the target area(s), as well as to follow the progression of the disease and / or the effectiveness of a treatment administered to the affected mammal.

In accordance with another aspect of the present invention, there is provided diagnostic kits comprising the compound of formula (I), a suitable delivery vehicle, reagents necessary for the radiolabeling of the compound with an appropriate radioisotope of iodine and instructions for use. The present invention further provides kits for preparing a radiolabeled diagnostic compound for use in radioimaging, comprising the compound of formula (I) and reagents necessary for the radiolabeling of the compound with an appropriate radioisotope of iodine. The kits may further contain emulsifying agents and core lipids necessary to prepare a microemulsion.

### BRIEF DESCRIPTION OF THE FIGURES

Figure 1 depicts the lipid staining (left panel) and autoradiogram (right panel) of a sample aorta from a group of rabbits maintained on a high cholesterol diet.
Figure 2 depicts the lipid staining (left panel) and autoradiogram (right panel) of a sample aorta from a group of rabbits maintained on a high cholesterol diet with anti-atherogenic drug treatment.
Figure 3 depicts the lipid staining (upper panel) and autoradiograms (lower panel) of aortae from ApoE/LDL receptor double knockout mice maintained on a normal Chowdiet.
Figure 4 provides a graphical representation of the relative biodistribution of ¹²⁵I-C2I in ApoE/ LDL receptor double knockout mice and control mice after intravenous injection of ¹²⁵I-C2I/ Ac LDL.
Figure 5 provides a graphical representation of the relative biodistribution of ¹²⁵IC2I in ApoE/ LDL receptor double knockout mice and control mice after intravenous injection of ^{I25}I-C2I / microemulsion.
Figures 6 and 7 depict the lipid staining (left panel) and phosphorimages (right panel) of aortae from ApoE/ LDL receptor double knockout mice injected with ¹²⁵I-C2I / Ac LDL.
Figures 8 and 9 depict the lipid staining (left panel) and phosphorimages (right panel) of aortae from ApoE/ LDL receptor double knockout mice injected with ¹²⁵I-C2I / microemulsion.
Figures 10 and 11 depict the lipid staining (left panel) and phosphorimages (right panel) of aortae from control mice.
Figure 12 depicts the receptor-mediated uptake of ¹²⁵I-DPIP / LDL by the cervical cancer cell line HeLa.
Figure 13 depicts the receptor-mediated uptake of ¹²⁵I-DPIP / LDL by the cervical cancer cell line SiHa.
Figure 14 depicts the receptor-mediated uptake of ¹²⁵I-DPIP / LDL by the cervical cancer cell line C-33A.

### DETAILED DESCRIPTION OF THE INVENTION

High quality radioimaging depends on maximum accumulation of a radiolabeled probe with a high specific radioactivity in the tissue to be imaged. This invention provides hydrolysis-resistant analogues of polyiodinated diacylglyceryl (IDG) ester for use as radiolabeled probes for diagnostic radioimaging. Like cholesteryl iopanoate (CI), these IDG analogues are based on the structure of native cholesteryl ester (CE) found in LDL particles, but are either completely resistant to lysosomal enzyme hydrolysis or are only slowly hydrolyzed. As such, these compounds become trapped inside target cells and the radiation they emit is thus localized to the targeted tissue. A further advantage of the IDG analogues of the present invention is the incorporation of between two and six iodine atoms into each molecule, thereby increasing the specific radioactivity and allowing higher quality images to be generated when using these probe molecules. In addition, the high specific activity of the compounds will help minimize the impact of the inevitable dilution of the radioisotope in the circulation.

### Definitions

The term "LDL receptor" (LDLR) as used herein, means a receptor capable of binding and internalizing LDL or modified LDL and includes both native LDL receptors and the family of scavenger receptors found in mammals.

The term "delivery vehicle" as used herein means a biocompatible carrier that is capable of transporting a synthetic compound, such as the radiolabeled probes of the present invention, in the bloodstream of a mammal. Examples include, but are not limited to, liposomes, antibody-linked conjugates, carbohydrate derivatives of the targeting compound microemulsions, LDL or chemically modified LDL (such as acetylated LDL or oxidized LDL).

Unless defined otherwise, all technical and scientific terms used herein have the same meaning as commonly understood by one of ordinary skill in the art to which this invention pertains.

The probe molecules of the present invention are analogues of IDG esters and have the following formula (I): 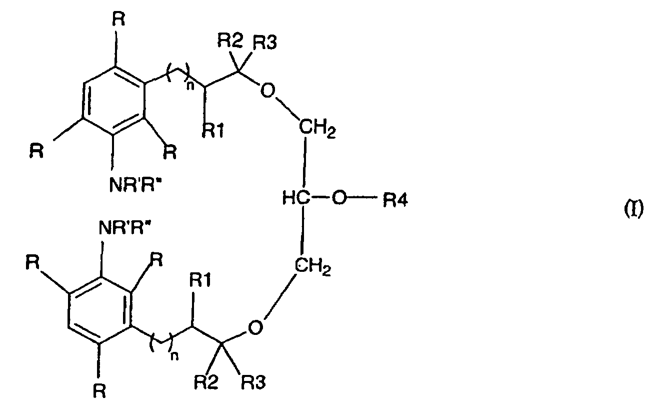 wherein:
n is an integer between 1 and 16;
each R group is independently H or I, wherein between 2 to 6 R groups must I;
R' and R" are independently H, (C1-C4 alkyl) or an amine protecting group;
R1 is H or (C₁-C₆) alkyl;
R2 is H;
R3 is H or when taken together R2 and R3 is =O; and
R4 is: 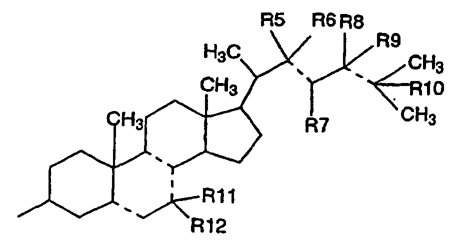 wherein:
   the bond - - - designates a single or a double bond;
   R5 is H, OR"' or R"', wherein R"' is (C₁-C₆) alkyl;
   R6 is H or absent;
   R7 is H or absent;
   R8 is H, (C₁-C₆) alkyl or (C₂-C₆) alkenyl;
   R9 is H or absent;
   R10 is H or absent;
   R11 and R12 are independently H, R"' or OR"', or when taken together R11 and R12 are =O;
   with the proviso that when R9 and R10 are absent and the bond - - - between carbon atoms bearing R9 and R10 is a double bond, then R8 is H or (C₁-C₆) alkyl; and that two adjacent bonds designated as - - - are not double bond at the same time.

### Preparation and Radiolabeling of the Probe Molecules

### I. Preparation of Compounds of Formula (I)

The compounds of formula **(I)** can be prepared as shown in Scheme I using a combination of conventional preparative steps and recovery methods known to those skilled in the art of organic synthesis. Starting compound (1) is coupled with dihydroxyacetone or its alkali metal salt, in the presence of an appropriate solvent to obtain compound (2). Compound (2) is then reduced using an alkali metal borohydride, such as sodium borohydride, to obtain a compound of structural formula (3). Compound (3) is further coupled with a halo-steryl derivative (5) in the presence of an appropriate base, such as sodium hydride, and an appropriate solvent to yield the compound **(I)**. Halo-steryl derivatives of structural formula (5) can be readily prepared from commercially available cholesterol and sterol derivatives of structural formula (4) using standard reactions known to a worker skilled in the relevant art for converting a hydroxy group to a halogen group, for example by reaction with either thionyl chloride or thionyl bromide.

A sterol derivative can be one of the commercially available sterols, or it can be a derivative of one of these sterols, which is readily prepared by standard synthetic techniques well-known to those skilled in the art. Commercially available sterols include, but are not limited to, β-sitosterol, dihydrocholesterol, 7-dehydrocholesterol, 22-hydroxycholesterol, 25-hydroxycholesterol, 3-cholesten-3β-ol-7-one, 5α-cholestane-3β-ol, 5α-cholest-7-en-3β-ol, 7β-hydroxycholesterol, campesterol, desmosterol, ergosterol, fucosterol, lanosterol and stigmasterol.

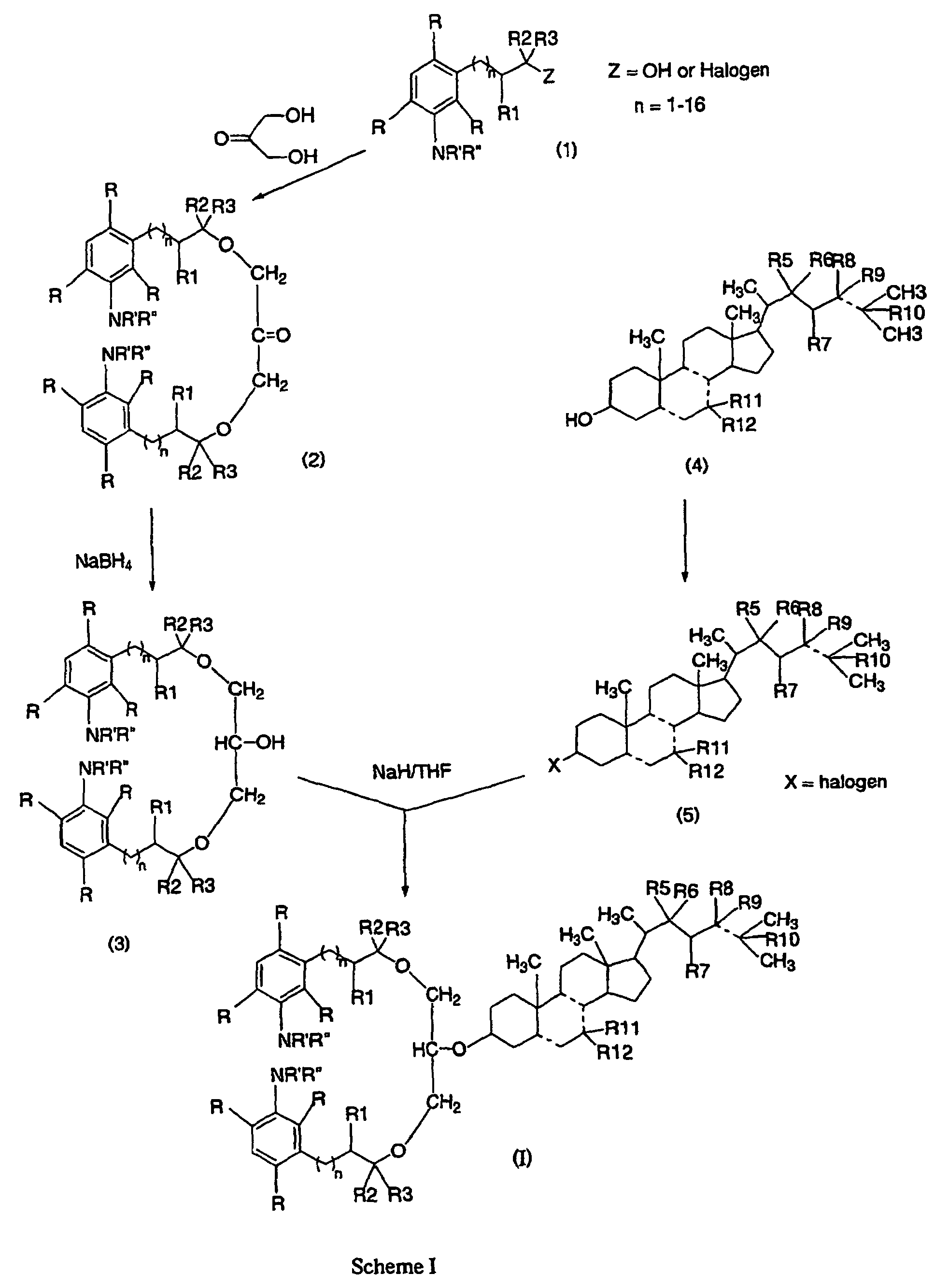

The starting compounds of formula (1) used in the preparation of probe molecules of structural formula (I) are either commercially available or can be prepared from other commercially available starting materials using standard reactions known to a worker skilled in the relevant art. Examples of some of the general methods for the preparation of compound of formula (1) are provided below in Schemes II - V (Methods A - D). These methods are merely illustrative and can be modified by those skilled in the relevant art for the production of compounds of formula (I) in accordance with the invention [Weichert, *et al., J Med. Chem.,* **29**:1675-1682 (1986); Weichert, *et al., J Med. Chem.,* **38**:636-646 (1995)].

### II. Preparation of Compounds of Formula (1)

### Method A

An example of a method to synthesize compounds of formula (1), wherein R2 and R3 when taken together is =O, is outlined in Scheme I. Bromoethyl ester (6) is reacted with triphenylphosphine to form a triphenylphosophonium salt (7), which on condensation with *m*-nitrobenzaldehyde in the presence of an appropriate base leads to the formation of compound (8). Compound (8) is reduced under catalytic hydrogenation conditions to give compound (9), which is then treated with iodine monochloride in the presence of acid to obtain compound (10). Compound (10) is then hydrolyzed to obtain compound (1), wherein R1 is H and Z is OR. Alternatively, compound (8) can be first treated with hydroxylamine sulphate in the presence of hydroxylamine-*O*-sulphonic acid to obtain compound (11), which is then subjected to standard α-alkylation conditions, followed by reduction under catalytic hydrogenation conditions to give compound (12). Compound (12) is treated with ICI in the presence of acid, followed by hydrolysis of the ester group to give compound (1), wherein R1 is other than H and Z is OH. The bromoethyl esters of structural formula (6) are either commercially available or can be prepared by standard reactions known to a worker skilled in the relevant art.

Compound (9) or compound (12) can alternatively be subjected to partial iodination conditions to obtain compounds of formula (1) that are mono- or di-iodo substituted.

A further alternative method of obtaining mono-iodinated compounds of formula (1) would be to subject compound (11) to α-alkylation, followed by iodination, hydrogenation and hydrolysis.

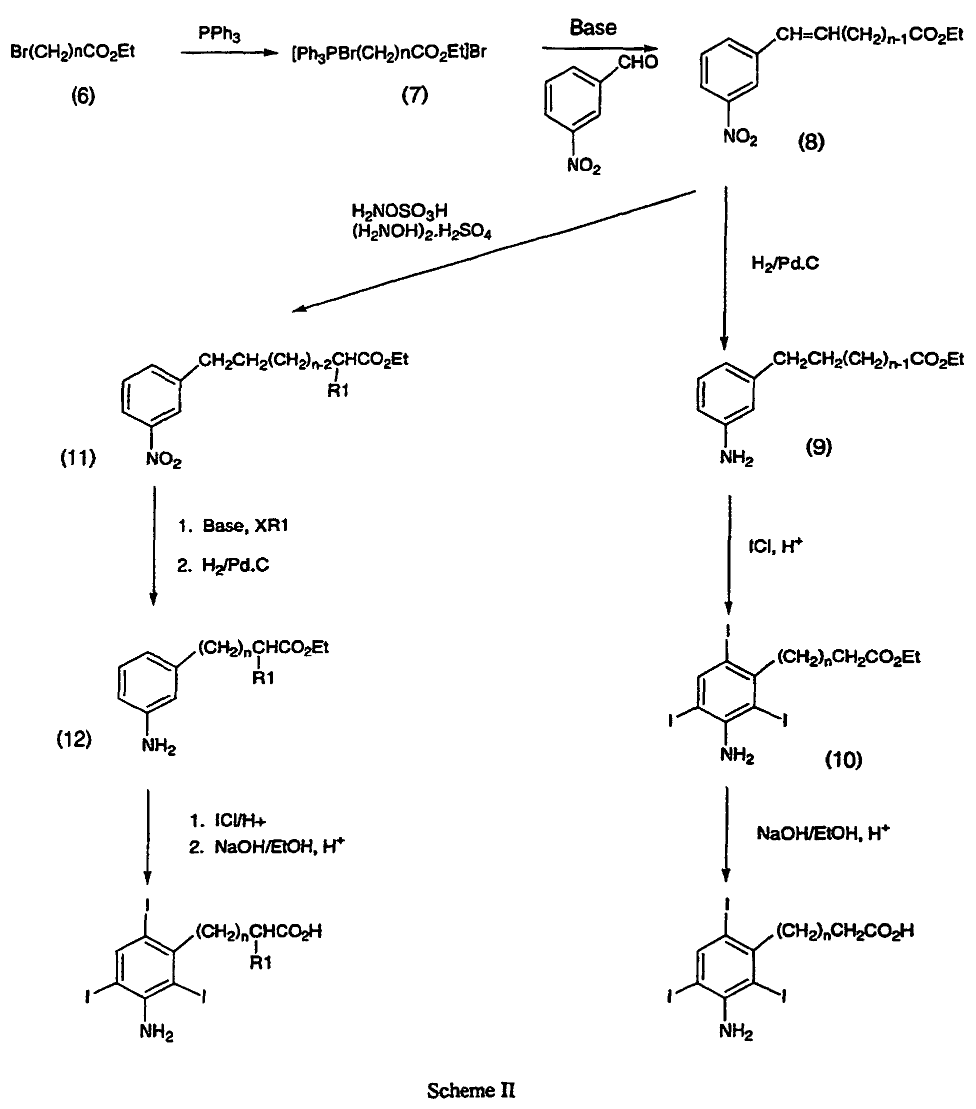

### Method B

Another method of synthesis of compounds of formula (1), wherein R2 and R3 when taken together is =O, is outlined in Scheme III and involves the reaction of a copper reagent (readily prepared from iodo ethyl ester (13) by reaction with zinc and dilithio salt of CuCN) with *m*-nitro benzyl bromide to yield compound of formula (14). Compound (14) can be converted to compound (1), wherein R1 is H and Z is OH, using standard reaction conditions as shown in Scheme II. Alternatively, compound (14) can be subjected to catalytic hydrogenation conditions to give compound (15), which can be then converted to compound (1), wherein R1 is other than H and Z is OH, as described in Scheme II. Iodoethyl esters of structural formula (13) are either commercially available or can be prepared from other commercially available starting materials by standard reactions known to a worker skilled in the relevant art.

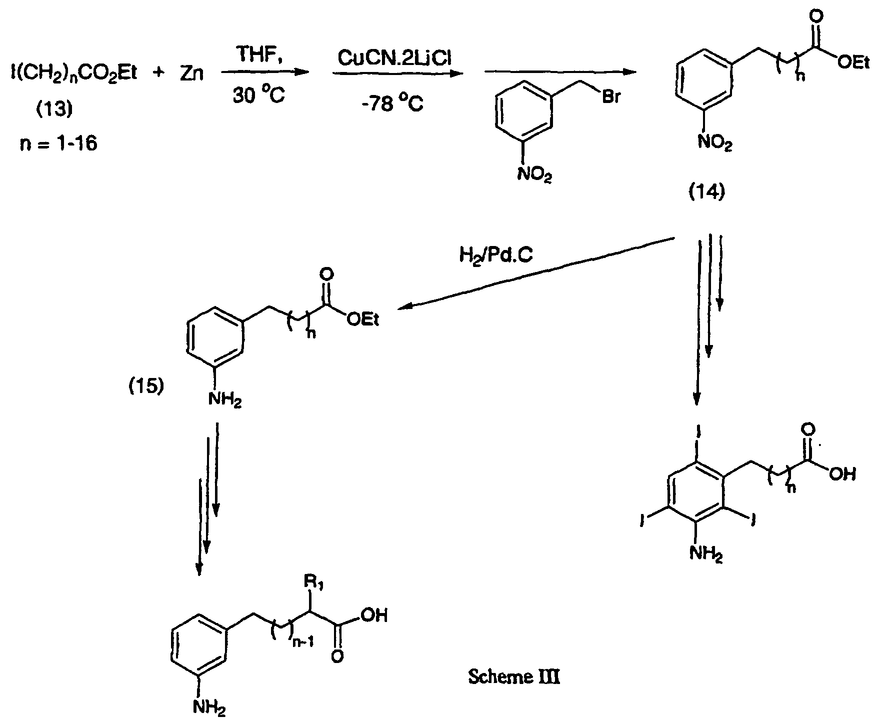

### Method C

Scheme IV outlines a further possible method of synthesis of compounds of formula (1), wherein R2 and R3 taken together is =O. *m*-nitro benzylbromide is reacted with triphenylphosphine to form the triphenylphosphonium salt (16), which on condensation with aldehyde (17) in the presence of an appropriate base and an appropriate solvent leads to the formation of compound (18). Compound (18) can then be subjected to catalytic hydrogenation, iodination and hydrolysis as outlined in Scheme II, to give compound (1), wherein R1 is H, Z is OH and n is 2-16. Alternatively, compound (18) can be first treated with hydroxylamine sulphate in the presence of hydroxylamine-0-sulphonic acid to give compound (21), which can then be subjected to standard α-alkylation conditions, catalytic hydrogenation, iodination and hydrolysis as outlined in Scheme II, yielding compound (1), wherein, R1 is other than H, Z is OH and n is 2-16. Aldehyde (17) is either commercially available or can be prepared by standard reactions known to a worker skilled in the relevant art.

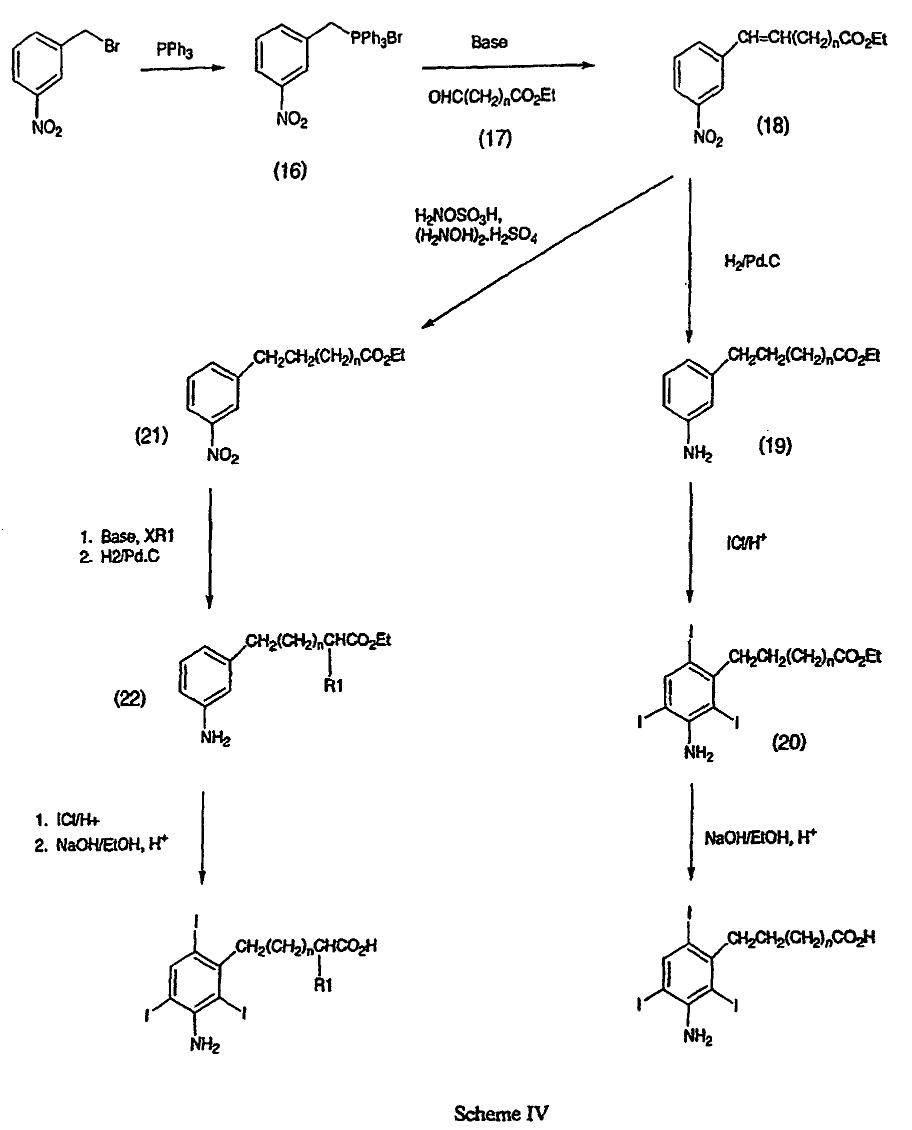

### Method D

Compounds of formula (1), wherein n = 1 and R1 is other than H, can also be prepared by the method described in U.S. Patent No.2,705,726 and outlined in Scheme V. According to this method, *m*-nitrobenzaldehyde is reacted with acid anhydride (23) in the presence of an appropriate base to yield compound (24). Compound (24) is reduced under catalytic hydrogenation conditions to obtain compound (25), which is treated with iodine monochloride in the presence of acid to yield compound (1), wherein n = 1, R1 is other than H and Z is OH. Acid anhydrides of formula (23) are either commercially available or can be prepared by standard reactions known to a worker skilled in the relevant art.

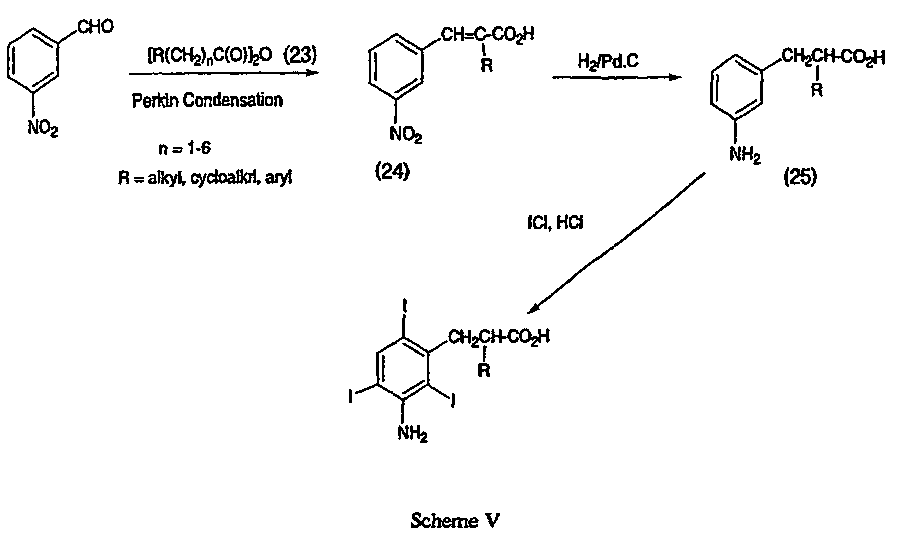

If required, compounds of formula (1), wherein R2 and R3 take together are =O and Z is OH, can be reduced with an alkali metal hydride, such as lithium aluminum hydride, as shown in Scheme VI to yield compounds of formula (1), wherein R2 and R3 are H and Z is OH.

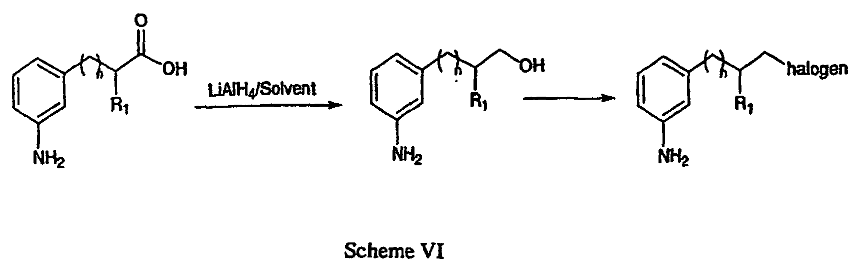

All compounds of formula (1), wherein Z is OH can be converted to compound of formula (1), wherein Z is halogen by standard reactions, such as reaction with thionyl halide or phosphorus halide, which are known to those skilled in the art. A worker skilled in the art will also recognize that compounds of formula (1), wherein R' and R" are other than H, can be prepared from compounds of formula (1), wherein R' and R" are H, by reaction with an amine protecting group or an alkyl halide using standard techniques.

It is to be understood that the present invention is considered to include stereoisomers as well as optical isomers, *e*.*g*. mixtures of enantiomers as well as individual enantiomers and diastereoisomers, which arise as a consequence of structural asymmetry in selected compounds of the present invention.

Without limiting the present invention to any particular mechanism of action, the inventors believe that the effectiveness of the probe molecules of the present invention may be due to the increased hydrophobicity provided by the steryl ether substituent at the 2-position on the glyceryl moiety (R4 in formula (I)). Those skilled in the art will appreciate that many chemical modifications may be readily made to this steryl moiety. It is therefore contemplated that the scope of the present invention encompasses analogues of the steryl moiety that retain the overall hydrophobicity of the molecule.

In one embodiment of the present invention the probe molecule is cholesteryl 1,3-diiopanoate glyceryl ether (C2I, Compound II), in which the sterol moiety is derived from cholesterol. '

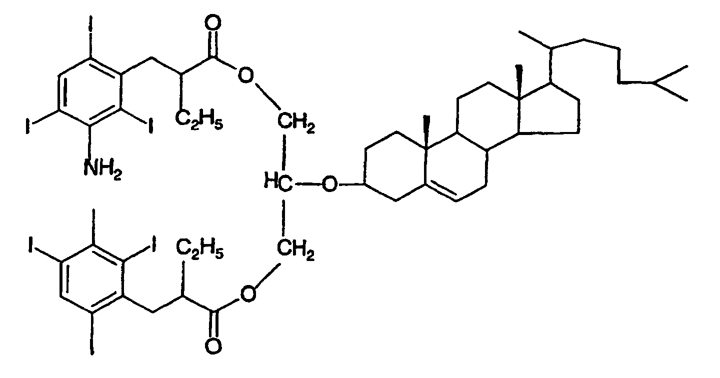

### III. Radiolabeling the Probe Molecules

The IDG analogues of the present invention can be readily radiolabeled with one of the clinically used radioisotopes of iodine, ¹²²I,¹²³I, ¹²⁵I or ¹³¹I. The long half-life of ¹²⁵I (60 days) makes it an exceptionally valuable isotope for both *in vitro* testing and for preliminary chemical and biological studies in animals. For clinical radioimaging, ¹²⁵I is usually replaced by ¹²³I or ¹³¹I, both of which have good imaging energy and shorter half-lives. ¹³¹I is readily available and economical, and is the radioisotope of iodine most frequently employed for organ-imaging in humans.

The IDG analogues of the present invention can be radiolabeled by isotope exchange methods, which are well-known to those skilled in the art. In general, isotope exchange involves reaction of the substrate in a "melt" with radioiodine using a suitable reaction medium and elevated temperatures. The dielectric constant of the molten reaction medium is sufficiently high to solubilize both reactants. For this purpose, the reaction medium can be, for example, benzoic acid, acetamide or pivalic (trimethyl acetic) acid [for example, see Weichert *et al., Appl. Radiat. Isot.,* **37**:907-913 (1986)]. Once the exchange reaction is complete, the radiolabeled compound can be purified by an appropriate technique, which can be readily determined by those skilled in the art.

In one embodiment of the present invention, C2I is radiolabeled with ¹²⁵I by radioiodine exchange in pivalic acid. In this procedure C2I is placed in a sealed vial to which freshly distilled tetrahydrofuran (THF) and aqueous Na¹²⁵I are added in succession. A gentle stream of nitrogen is then applied to evaporate the solvents and solid pivalic acid is added to the dry residue. The vial is partially immersed in an oil bath pre-heated to 155-160°C. The isotope-exchange reaction is essentially complete after 1-2 h at this temperature, after which time the reaction vial is allowed to cool and the contents are dissolved in dry THF. The reaction can be monitored by thin-layer chromatography using a small sample removed at this point and visualized under ultraviolet light and by autoradiography. The radiolabeled C2I is then purified on a silica gel-60 column, and the purity is determined by HPLC analysis. By this procedure, radiochemical purity of the final compound usually exceeds 95%.

### Methods of Delivery of Radiolabeled Probes

Targeted delivery of the radiolabeled probes of the present invention can be achieved by a number of methods. Such methods generally involve incorporation into a suitable delivery vehicle and include, but are not limited to, incorporation into liposomes, microemulsions, LDL or chemically modified LDL (such as acetylated LDL or oxidised LDL).

Once radiolabeled, the IDG analogues of the present invention are designed to function as radioimaging probes that target to regions of the body where high concentrations of LDL receptors are found. These regions include, but are not limited to, atherosclerotic lesions, hepatocytes, tumour cells, neural cells and areas of macrophage accumulation. Those with skill in the art will appreciate the role of LDL receptors is not wholly understood and knowledge of this role is still evolving. Future research may discover new tissues, healthy or diseased, in which high concentrations of LDL receptors are found and, therefore, new areas where the probes of the present invention may be applied for diagnostic purposes.

It is envisioned that other compatible bioactive agents for therapeutic and / or diagnostic purposes may be incorporated with the radiolabeled probes of the present invention into the delivery vehicles for targeted delivery to regions of the body where high concentrations of LDL receptors are found.

### Microemulsion-based delivery systems

The radiolabeled probes of the present invention can be incorporated into synthetic microemulsions for targeted delivery.

To prepare synthetic microemulsions, one or more emulsifying agents are mixed with one or more core lipid components in which the radiolabeled probe is dispersed. Emulsifying agents for this purpose are generally phospholipids of natural, synthetic or semi-synthetic origin. Examples of emulsifying agents that can be used to prepare suitable microemulsions include, but are not limited to, L-α-dipalmitoyl phoshatidylcholine (DPPC), DL-α-dipalmitoyl phoshatidylethanolamine (DPPE), dioleoyl phosphatidylcholine (DOPC), cholesterol, soy lecithin, egg phosphatidylcholine and egg lecithin. In one embodiment of the present invention DPPC and DPPE are used as emulsifying agents in the formation of the microemulsion.

The radiolabeled probes of the present invention are typically dispersed in a core lipid component for incorporation into the microemulsions. In general, core components are oils of animal or vegetable origin, or synthetic or semi-synthetic oils. Examples of these oils include, but are not limited to, triolein, squalene, fish oils, seal oil, soya bean oil, canola oil, cottonseed oil, safflower oil and corn oil. Factors that need to be considered when determining the suitability of a core lipid for use in microemulsion formation include the level of incorporation of the hydrophobic radiolabeled probe into the microemulsion and the biocompatibility of the oil.

In one embodiment of the present invention, harp seal oil is used as the core lipid resulting in high levels of incorporation of the radiolabeled probe into synthetic microemulsions. Seal oil has high fluidic characteristics, and has been found to contain substantial levels of highly unsaturated triglycerides, which may allow for greater solvation of the hydrophobic compounds of the present invention than other oils.

Synthetic lipid emulsions with an average particle size in the range of 50 - 200 nm have been shown to follow similar metabolic routes to human chylomicron remnants, probably due to their similar particle size [Weichert *et al., J. Med. Chem.,* **38**: 636 - 646 (1995)]. Since chylomicron remnants are recognized by LDL receptors, the use of microemulsions as delivery vehicles provides a means of selectively targeting radiolabeled probes to areas that possess high concentrations of LDL receptors. Microemulsions with an appropriate average particle size (*i.e.* 50 - 200 nm in diameter) may be prepared by a number of techniques well-known in the art, for example, by homogenization, sonication or microfluidization. A variety of suitable mechanical devices to achieve the preparation of microemulsions are commercially available.

In one embodiment of the present invention, the radiolabeled probe ¹²⁵I-C2I is incorporated into microemulsions with an average particle size of 50 - 70 nm in diameter. The microemulsions are prepared by dissolving DPPC, DPPE, seal oil and ¹²⁵I-C2I in chloroform, drying the mixture under nitrogen and then resuspending the residue in saline. The resuspended mixture is sonicated for 2 hours with cooling under a stream of nitrogen, then submitted to ultracentrifugation at 40 000 revolutions per minute (rpm) for twenty hours. The top layer of the centrifugate containing the microemulsion is removed and passed through a miniextruder to select the correct average particle diameter.

Synthetic microemulsions can also be prepared by microfluidization techniques [for example, see Weichert *et al., J. Med. Chem.,* **38**:636-646 (1995)]. In this case, the microemulsion is formed as two fluidized streams (containing core lipids, emulsifying agents and probe molecules), which interact and / or collide at high velocities in a interaction chamber. Microfluidizer processors are available commercially that are driven by air or nitrogen and operate at internal pressures of 20 000 psi with a throughput of 300 - 500 µl per minute. Within the microfluidizer, the microemulsion can be continuously recycled through a closed-loop system, thus producing uniform particle sizes.

Homogenization techniques can also be used to prepare crude microemulsions with an additional high energy microfluidization or sonication step to prepare the final microemulsion [for example see, U.S. Patent No.6,126,946].

### LDL-Based delivery systems

LDL suitable for use as delivery vehicles may be prepared from human plasma. Separation of LDL from the other lipoproteins present in plasma can be achieved by methods well-known in the art, for example, by sequential density ultracentrifugation [see *Methods in Enzymology,* **128**: 150-209]. For use as a delivery vehicle, the LDL may be further modified, for example by acetylation or oxidation. Standard techniques for modification of lipoproteins are well-known to those skilled in the art, for example, see Basu and Goldstein, *Proc. Natl. Acad. Sci. USA,* **73**: 3178-3182 (1976).

The radiolabeled probes of the present invention can be loaded into the LDL particles by one of several methods known in the art. Examples of loading techniques include, but are not limited to, direct diffusion, reconstitution, detergent solubilization, use of organic solvents, use of donor particles such as microemulsions, and use of transfer proteins such as insect lipid transfer protein (iLTP).

In one embodiment of the present invention, a microemulsion is used as a donor particle to load the radiolabeled probes into LDL. Synthetic microemulsions, such as those described above, can be used to provide high levels of incorporation of the radiolabeled probes of the present invention into LDL. This use of microemulsions can help to overcome some of the drawbacks associated with direct diffusion and detergent solubilization, such as low levels of incorporation or denaturation of the Apo B component of the LDL. The radiolabeled probes are readily transferred from the microemulsion to the LDL by incubation at physiological temperature, with subsequent separation of the loaded LDL from unloaded LDL by ultracentrifugation.

In another embodiment of the present invention, the radiolabeled probes are loaded into LDL by catalysis using a lipid transfer protein. Transfer proteins suitable for this use include, but are not limited to, human CE transfer protein (CETP) and tobacco hornworm (*Manduca sexta*) lipid transfer protein (iLTP). Transfer proteins usually mediate the movement of hydrophobic materials associated with lipoproteins in the circulatory system. Other substrates may also be transferred by these proteins, for example, iLTP has been shown to transfer diacylglycerol (DG), triacylglycerol, phospholipids, cholesterol, CE and hydrocarbon wax [Ryan *et al., J. Biol. Chem.,* **265**:10551-10555 (1990); Liu and Ryan, *Biochim. Biophys. Acta,* **1085**:112-118 (1991)]. Methods of isolating and purifying. iLTP have been published [Ryan, *J. Lipid Res.,* **31**:1725-739 (1990); Liu and Ryan, *Biochim. Biophys. Acta,* **1085**:112-118 (1991)].

In one embodiment of the present invention, the radiolabeled probe is loaded into LDL by iLTP catalysis. LDL can be loaded using iLTP by dissolving the radiolabeled probe in an appropriate solvent, removing the solvent under nitrogen, and resuspending the residue in a small quantity of wetting agent. LDL is then added, and the mixture is incubated for an appropriate length of time, typically about 4 hrs, at 37°C. After, incubation, the mixture is centrifuged at an appropriate density to allow the LDL / probe complex to float to the top of the centrifuge tube, and thus separate it from the unbound drug and iLTP. The LDL / probe complex fractions are then collected and dialyzed to remove any remaining unbound probe molecules. The amounts of radiolabeled probe associated with the LDL can be determined by standard techniques, for example, by using a gamma counter.

In another embodiment of the present invention, a combination of the use of microemulsions as donor particles with the use of iLTP as a catalyst is used to efficiently load the LDL or modified LDL with the radiolabeled probe.

### In vitro Testing of the Radiolabeled Probes

Once the radiolabeled probes of the present invention have been incorporated into an appropriate delivery vehicle, the level of incorporation can be determined by measuring the amount of radioactivity associated with the loaded delivery vehicle by standard techniques, for example, by gamma counter or liquid scintillation counting.

In one embodiment of the present invention, AcLDL is loaded with ¹²⁵I-C2I and the radioactivity and protein content of the complex is determined by standard techniques. The integrity of the apo B-100 protein is then assessed by analyzing samples by sodium dodecyl-polyacrylamide gel electrophoesis (SDS-PAGE) and electron microscopy as previously described [Deforge, *et al., Nucl. Med. Biol.,* **19**:775-782 (1992)].

Cellular uptake of the radiolabeled probes can be tested *in vitro* in an appropriate cell-line. For example, hepatic cell-lines or scavenger receptor-rich cell-lines such as J774A1 can be used. Alternatively, since it has been demonstrated that tumour cells have increased expression of LDL receptors [for example, see Ho, *et al., Blood,* **52**:1099-1104 (1978); Chen and Hughes-Fulford, *Int. J. Cancer,* **91**:41-45 (2001); Xiao, *et al., Radiat*. *Res.,* **152**:250-256 (1999)], a cancer cell-line may be used. The selected cell-line is cultured by standard techniques and the radiolabeled probe in the chosen delivery vehicle is added to the culture medium. The cells are incubated for an appropriate length of time, typically about 4 hours, then the medium is removed and the cells washed extensively to remove external radioprobe. The cells are lysed and the amount of radiolabeled probe taken up by the cells can be determined by measurment of the radioactivity associated with the lysed cells by standard methods.

### In vivo Testing of the Radiolabeled Probes

The radiolabeled probes of the present invention may be tested for the visualization of target tissues *in vivo* with an appropriate animal model. The radiolabeled probe can be introduced into the animal by injection and the biodistribution of the probe determined by standard methods [for example, see Xiao *et al., Pharm. Res.,* **16**:420 - 426 (1999)]. The radiolabeled probes can be injected into the test animals in one of the delivery vehicles described above. The animals can be sacrificed at appropriate times post-injection and the tissues and / or organs of interest removed. Analysis of the amount radioactivity associated with the tissue / organ can then be determined by standard techniques, for example by gamma counter or autoradiography, and used to determine the level of accumulation of the radiolabeled probe. Kinetic studies can be conducted to determine the optimal time post-injection for radioimages to be taken.

For example, the radiolabeled probes of the present invention can be used to visualize atherosclerotic lesions in animal models. Suitable atherosclerosis animal models include, but are not limited to, B6, 129 ApoE^{*tm1Unc*} Ldlr^{*tm1Her*} (ApoE/LDLR) transgenic mice, cholesterol-fed rabbits or miniature swine. If necessary, atherosclerotic plaques can be introduced by dietary, chemical and / or surgical treatment. Alternatively, the radiolabeled probes of the present invention can be used to visualize tumours in animal models. Tumours can be induced by techniques well-known in the art, for example, by exposing the animals to carcinogenic chemicals or by transplanting cultured tumour cells into the appropriate site / organs. The relative distribution of the injected radiolabeled probes in the tumour tissue and the surrounding healthy tissue will provide an indication of the specificity of the probe.

In one embodiment of the present invention, the efficacy of the radiolabeled probe for the visualization of atherosclerotic lesions is tested in a mouse model. The radiolabeled probe is injected into the tail vein of B6, 129 ApoE^{*tm1Unc*} Ldlr^{*tm1Her*} (ApoE/LDLR) transgenic mice, which are an accepted atherosclerosis model. The mice are sacrificed 24 hours postinjection and a fixative is infused into the heart. Biodistribution of the probe is determined by sampling different organs with a gamma counter. The accumulation of probe in the blood vessels is then visualized by removal of the aortae, staining with an appropriate dye, such as Sudan IV, to visualize the lesioned areas and subsequent exposure to a phosphor screen.

### Applications

The present invention provides hydrophobic radiolabeled probes that may be used for the visualization of tissues or regions in a mammal where there are high concentrations of LDL receptors by radioimaging techniques. It is envisioned that the probe may be used for diagnostic purposes to determine the presence and / or extent of a disease that affects the target area(s), as well as to follow the progression of the disease and / or the effectiveness of a treatment administered to the affected mammal. In one embodiment of the present invention, the mammal is a human.

The radiolabeled probes of the present invention may be administered to a mammal in one or more of the targeted delivery vehicles described above. In one embodiment of the present invention, the radiolabeled probe, incorporated into either AcLDL or a microemulsion, is administered in a single unit injectable dose. The unit dose to be administered will depend on the application and individual mammal to which it is being administered. Dose ranges for clinical radioimaging can be readily determined by those skilled in the art. Generally, the unit dose to be administered has a radioactivity of about 0.01 mCi to about 100 mCi, preferably 1 mCi to 20 mCi. The solution to be injected at unit dosage is from about 0.01 mL to about 10 mL.

After intravenous administration of the radiolabeled probe to a patient, radioimaging *in vivo* can take place at a time within the range of a few minutes to a few hours post-injection. Localization of the radiolabeled probe is detected by conventional clinical radioimaging techniques.

The radiolabeled probes of the present invention are designed to target and provide a means of visualizing regions of the body containing LDL receptors, such as liver cells, neural cells and tumor cells. In one embodiment, the probes are designed to visualize regions of the body containing elevated levels of LDL receptors, such as atherosclerotic lesions. It is envisioned, therefore, that the probes can be used as diagnostic agents. In one embodiment the probes are used as diagnostic agents for diseases and conditions such as atherosclerosis, stroke, cirrhosis, hepatitis, Alzheimer disease, glomerulosclerosis, ataxia with Vitamin E deficiency, liver tumors, and cancer. It is further envisioned that the probes of the present invention may be used for monitoring purposes, for example to monitor the progress of a disease state in a patient, remission of a disease state subsequent to surgery and / or drug therapy, or hepatic function after a liver transplant.

### Diagnostic Kits

The present invention further provides for diagnostic kits containing the radiolabeled probes of the present invention. Individual components of the kit would be packaged in separate containers and, associated with such containers, can be a notice in the form prescribed by a governmental agency regulating the manufacture, use or sale of pharmaceuticals or biological products, which notice reflects approval by the agency of manufacture, use or sale for human administration. The kit would further contain instructions describing the appropriate method of use of the components. To maximize the shelf-life of the kits, the probes can be provided unlabeled and the kit can further contain the reagents and protocols necessary for radiolabelling of the probe molecules with the appropriate radioisotope.

To gain a better understanding of the invention described herein, the following examples are set forth. It should be understood that these examples are for illustrative purposes only. Therefore, they should not limit the scope of this invention in any way. In particular, the methods of synthesis and radiolabelling are merely illustrative and can be modified by those skilled in the art to produce functional analogues of the radiolabeled probes described below.

### EXAMPLES

### GENERAL

Thin layer chromatography (TLC) was carried out on silica gel 60, F-254 polyethylene-backed plates (Fisher Scientific) and visualized under UV light. Column chromatography was performed on silica gel-60 (230-400 mesh) (Aldrich). Radioactivity was measured using a CKB-C Wallac 1277 Gammamaster automatic gamma counter. Sodium dodecyl sulfate polyacrylamide gel electrophoresis (SDS-PAGE) was performed on a Mini-PROTEIN® II Electrophoresis Cell. Image analysis was performed on a BIOQUANT™ System IV. Carrier-free aqueous Na¹²⁵I was purchased from NEN Life Science (Boston, MA). Pivalic acid was obtained from Aldrich. Bio-Rad protein assay standards were purchased from Bio-Rad Laboratories (Hercules, CA). L-α-dipalmitoyl phosphatidylcholine (1,2-dihexadecanoyl-*sn*-glycero-3-phosphocholine, DPPC) and DL-α-dipalmitoyl phosphatidylethanolamine (1,2-dihexadecanoyl-*rac*-glycero-3-phosphoethanolamine, DPPE) were purchased from Sigma Chemical Co. (Oakville, ON, Canada). Seal oil was obtained from Terra Nova Fishery, Newfoundland, Canada.

### EXAMPLE 1: Preparation of 1,3-dihydroxypropan-2-one 1,3-diiopanoate (DPIP), glyceryl 1,3-diiopanoate (GIP) cholesteryl 1,3-diiopanoate glyceryl ether (C2I) [Scheme VII]

1,3-dicyclohexylcarbodiimide (DCC, 9.8g, 47.5 mmol) was added to a rapidly stirring suspension of dihydroxyacetone dimer (1.97g, 21.9 mmol), iopanoic acid (25.0g, 43.2 mmol), and a catalytic amount of 4-dimethylaminopyridine (DMAP, 500mg) in dry CH₂Cl₂ (200 ml). The reaction mixture was allowed to stir under N₂ for 66 h, after which it was diluted with CH₂Cl₂, and subsequently filtered to remove precipitated 1,3-dicyclohexylurea (DCU). The filtrate was washed with 0.5 N HCl (2x), saturated aqueous NaHCO₃ (2x), H₂O, and brine then dried with anhydrous MgSO₄. The solvent was removed *in vacuo* and the residue was triturated with anhydrous ether to precipitate any remaining traces of DCU. The resulting residue was re-crystallized from acetone to give DPIP as an off-white powder. Yield 85%.

A stirred suspension of DPIP (4.3g, 3.6 mmol) in a mixture of tetrahydrofuran (THF, 30ml), benzene (8 ml), and water (2ml) was cooled to 5°C in an ice bath and treated with neutral NaBH₄ (204mg, 5.4 mmol). The reaction mixture was stirred an additional 30 min at 5°C then treated with glacial acetic acid (0.3ml) to destroy excess borohydride. The resulting solution was diluted with CH₂Cl₂ (250ml) and extracted with saturated aqueous NH₄Cl (2x), H₂O, and brine and dried with anhydrous MgSO₄. Removal of solvent *in vacuo* afforded semi-pure product, which was further purified on silica gel column eluted with CHCl₃/hexane/ethyl acetate (5:3:2). The combination of the appropriate fractions and removal of solvents afforded GIP as a yellow solid. Yield 75%.

Sodium hydride (10mg, 0.4mmol) in 5ml anhydrous THF was added to a stirred suspension of GIP (299mg, 0.25mmol) and cholesteryl bromide (116.6mg, 0.26mmol) in 3ml anhydrous THF. The mixture was set overnight at room temperature. Then 8ml water was added, and the solution extracted with ether. The ether extracts were combined and residual water was removed by MgSO₄ addition. Subsequent emoval of the ether *in vacuo* afforded a semi-pure solid, which was purified by column chromatography on silica gel eluted with CHCl₃. Combination of the appropriate fractions and removal of the solvent *in vacuo* afforded C2I as white crystals. Yield 75mg (20%).
C2I data:
IR (cm⁻¹): 3500,3380 (-NH₂), 2950, 2840 (-CH₃), 1480 (C=C), 1740 (-C=O), 1380 ((CH₃)₂-CH-), 1600,920 (Ar)
¹H NMR (ppm): 8.1(s, 2H, Ar-H), 5.4 (s, 1H C=C-H), 4.8 (s, 4H, NH₂), 4.1 (m, 4H, CH₂), 3.7 (m, 2H, -O-CH), 3.4-3.2 (m, 4H, CH₂), 2.8 (m, 2H, CHCO₂), 2.2-1.6 (m, 22H, CH₂, CH), 1.1-1.3 (m, 10H, CH₂), 0.9 (d, 15H, CH₃), 0.7 (s, 6H, CH₃).

### EXAMPLE 2: Radiolabelling C2I with ¹²⁵I by radioiodine exchange in pivalic acid.

### General procedure

The compound to be radioiodinated (1-5 mg) was placed in a 2-mL serum vial which was then sealed with a Teflon-lined rubber septum and an aluminum cap. Freshly distilled tetrahydrofuran (THF, 100-200 µL) and aqueous Na¹²⁵I (10-50 µL) were added in succession via a microlitre syringe and the vial was gently swirled to dissolve the contents and ensure homogeneity. Inlet and outlet cannuli were inserted into the vial through the septum and a gentle stream of nitrogen was applied to evaporate the solvent. When the residue appeared dry, the seal was removed and solid pivalic acid (5-20 mg, dried by azeotrope with toluene

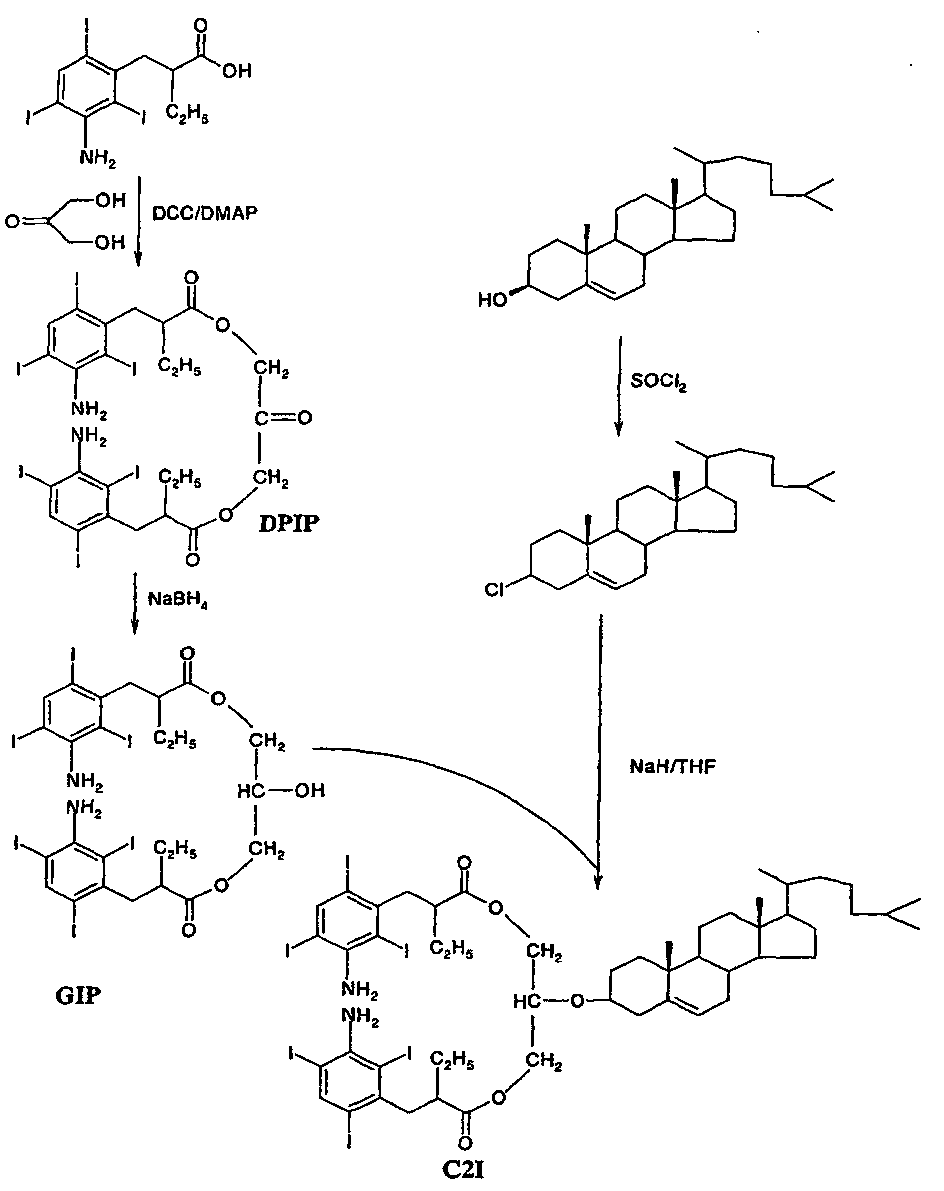

Scheme VII. Preparation of cholesteryl 1,3-diiopanoate glyceryl ether (C2I). and distilled under nitrogen) was added. The vial was resealed and partially immersed in an oil bath preheated to 155 - 160°C. When the isotope-exchange reaction was essentially complete (usually 1 - 2 h), the reaction vial was allowed to cool, then dry THF (200µL) was added with a glass syringe and the vial swirled gently. At this point a sample (1-2µL) was removed with a 10µL syringe for analysis by thin layer chromatography (TLC). The remaining contents were transferred to the top of a silica gel-60 column (1 x 10 cm) and subsequently eluted with the appropriate solvent system. If necessary, especially when labelling polar compounds, excess pivalic acid can be removed prior to chromatography by inserting a disposable syringe containing granulated charcoal as a trap into the reaction vial while heating and allowing the pivalic acid to distill into the trap. When eluting the column, a survey meter probe was placed at the outlet of the column to serve as a radiodetector. Fractions were collected and the radiochemical purity of each was monitored by TLC using ultraviolet (UV) and radioactivity detection: The appropriate fractions were combined and the solvent was removed with a gentle stream of nitrogen. HPLC analysis of the final compound confirmed both chemical (UV) and radiochemical (radioactivity) purity. The UV and the radioactivity values were plotted simultaneously using a two-pen strip chart recorder allowing calculation of the specific activity of the radiolabeled compound to be determined by comparison of the UV and radioactivity peak areas with standard calibration curves. Further radiopurification can be undertaken if desired. In all cases, the radiochemical purity of the final compounds exceeded 95%.

### EXAMPLE 3: Preparation of C2I/lipid microemulsions

### By Sonication

**I.** L-α-dipalmitoyl phosphatidylcholine (DPPC, 8mg); DL-α-dipalmitoyl phosphatidylethanolamine (DPPE, 8mg), seal oil (20mg), C2I (10mg) and ¹²⁵I-C2I (0.05mg) were dissolved in chloroform, dried in a gentle stream of nitrogen and then resuspended in 10ml saline solution at 25°C. The suspension was sonicated for 2 hours with a Virosonic Cell Disrupter (Model 16-850) at 40 - 50 watts under a constant stream of nitrogen while being cooled in a salt-ice water bath at -10 °C. The mixture was then centrifuged at 45 000 rpm for 8 hours at 4°C. The top creamy portion of the centrifuged mixture was collected and passed through a miniextruder fitted with a 0.05µm pore size polycarbonate membrane.
**II.** C2I microemulsions were prepared using triolein, canola oil, squalene, and seal oil, respectively, as the core lipid component. DPPC (12 mg), DPPE (8 mg), triolein, seal oil (20 mg) and ¹²⁵I-C2I (10 mg) were dissolved in chloroform, dried with a gentle stream of nitrogen, and resuspended in 10 mL of saline at 25ºC. The suspension was sonicated for 2 hours using a Virosonic Cell Disrupter Model 16-850 at 40-50 watts with cooling in a salt-ice-water bath at -10 °C under a nitrogen stream. The mixture was then centrifuged at 40 000 rpm for 20 hours at 4 °C using a Beckman SW41 rotor and a Beckman L5-65 ultracentrifuge. The microemulsion, which floated to the top of the centrifuge tubes, was collected and subjected to EM and image analysis. The amount of C2I in the microemulsion was determined using a CKB-WALLAC 1277 GAMMAMASTER automatic gamma counter.

### EXAMPLE 4: Preparation of AcLDL

Fresh human plasma was obtained from the Canadian Red Cross (St. John's, Canada) and specific preservatives were added [Edelstein and Scanu, *Methods in Enzymology,* **128:** 151-155 (1986)]. LDL was isolated by sequential ultracentrifugation of fresh human plasma at 40 000 rpm for 24 - 40 hours at 8°C using a Beckman L8-M ultracentrifuge and a 60Ti rotor as previously described [*Methods in Enzymology,* **128**: 150-209 (1986)]. All LDL preparations were dialyzed at 4°C overnight against a buffer containing 0.3 mM EDTA, 150 mM NaCl and 50 mM Tris (pH 7.4). Protein concentrations were determined by the Bradford Assay with bovine serum albumin (BSA) as standard and read with a BIO-RAD Model 550 Microplate Reader. LDL was stored at 2 - 8°C for a maximum of two weeks before use.

Acetylation of the isolated LDL was performed according to Basu and Goldstein [*Proc. Natl. Acad. Sci. USA,* **73**: 3178-3182 (1976)]. Basically, a mixture was prepared containing 5 ml of dialyzed LDL, 5 ml of 0.85% NaCl and 10 ml of saturated sodium acetate solution, and placed on ice. Acetic anhydride (70µl) was added in 10µl aliquots with stirring every 5 minutes. The resulting AcLDL was dialyzed at 4°C overnight against a buffer containing 0.3 mM EDTA, 150 mM NaCl and 50 mM Tris (pH7.4). Protein concentrations were determined as described above.

### EXAMPLE 5: Loading C2I into AcLDL from microemulsions

The ¹²⁵I-C2I microemulsions were incubated with AcLDL at 37°C for 24 hours at a molar ratio of C2I : AcLDL of 1000:1. AcLDL loaded with ¹²⁵I-CZI (¹²⁵I-C2I/AcLDL) was then separated from the microemulsion by ultracentrifugation. The C2I content in the ¹²⁵I-C2I/AcLDL particles was determined by gamma counting.

In order to assess the integrity of the apo B-100 protein of the ¹²⁵I-C2I/AcLDL particles, samples were analyzed by SDS-PAGE and EM as described previously [Deforge, *et al., Nucl. Med. Biol.,* **19**:775-782 (1992)].

C2I can also be incorporated into AcLDL using direct diffusion or detergent solubilization techniques. Published protocols for direct diffusion [Shaw, *et al., Ann. N. Y. Acad. Sci.,* **507**:252 - 271 (1987)] and detergent solubilization [Deforge, *et al., Nucl*. *Med. Biol.,* **19**:775 - 782 (1992)] were used with minor modifications. In brief, for direct diffusion, C2I was dissolved in chloroform in a glass vial then dried under a stream of nitrogen to produce a thin film on the wall of the vial. AcLDL was added and the vial was incubated at 37°C for 24 hours. For detergent solubilization, C2I was dissolved in saline in the presence of Tween-20 (<3%), then incubated with AcLDL at 37°C for 24 hours. The molar ratio of C2I to AcLDL used was the same as indicated above for the microemulsions.

### EXAMPLE 6: Detection of atherosclerotic lesions in rabbit and mouse atherosclerosis models using ¹²⁵I-C2I/AcLDL

Twelve male New Zealand White rabbits (2.5 - 4.7 kg) were purchased from Charles River Ltd. (Montreal, PQ, Canada) and randomly divided into three groups. For twelve weeks the groups were maintained on the following diets: one group on normal rabbit chow (n=4, control group), one group on rabbit chow supplemented with 1% cholesterol (n=4, cholesterol group) to induce the development of early stage atherosclerotic lesions, and the third group on rabbit chow supplemented with 1% cholesterol plus an anti-atherogenic agent (10mg / kg / day). After 12 weeks, the ¹²⁵I-C2I / AcLDL preparation was injected via the marginal ear vein in all three groups of rabbits at a dose of 15 µCi /kg. Twenty-four hours postinjection the rabbits were sacrificed and tissues, including liver, spleen, lung, kidney, adrenal gland, blood and aortae, were sampled to determine the distribution of radioactivity using a gamma counter. The aortae of the rabbits were washed, fixed and stained with Sudan IV dye to identify the atherosclerotic lesions. The red Sudan IV lipid stain is an indication of lipid-laden foam cells, a hallmark of early atherosclerosis. Histological examination of the aortae was conducted, followed by autoradiograpy using either X-ray films or a phosphor imager.

Similar experiments were conducted in B6, 129 ApoE^{*tm1Unc*} Ldlr^{*tm1Her*} (ApoE/LDLR) transgenic mice, which are an accepted atherosclerotic model. These mice carry a double mutation (in the apoE apolipoprotein gene and the LDL receptor gene) and can develop atherosclerotic lesions spontaneously. The strain has a similar lipoprotein profile to that of the hyperlipidemia patients. The B6, 129 ApoE^{*tm1Unc*} Ldlr^{*tm1Her*} transgenic mice were purchased from the Jackson Laboratory (Bar Harbor, Maine).

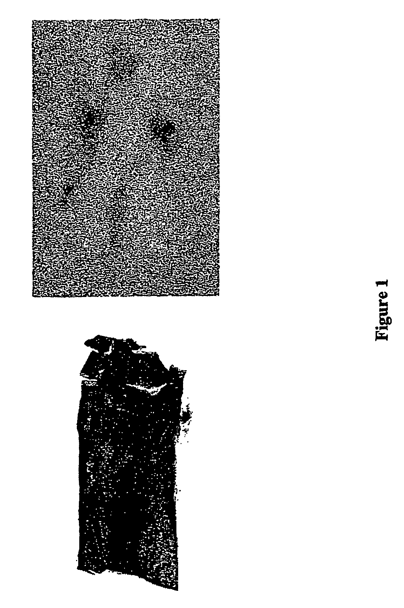

The left panel of Figure 1 shows the Sudan IV stained aortae from the cholesterol group of rabbits. The lesion area in these aortae corresponded to about 5% of the whole vessel. The most severe lesion region was the ascending aorta at the aortic arch. Blood flow through this section of aorta is turbulent due to the curve of the aortic arch and the three large branches. The highest incidence of human atherosclerosis also occurs in this region of the aorta.

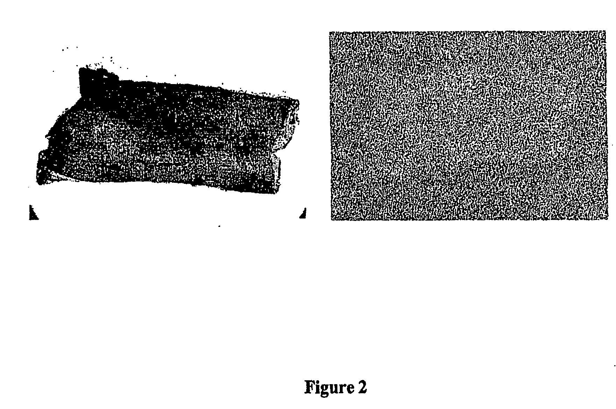

Autoradiographs of aortae removed from the cholesterol-fed rabbits are shown in the right panel of Figure 1. The images demonstrate the regions of ¹²⁵I-C2I / AcLDL accumulation. Superimposition of the autoradiographs on the stained aorta indicates that ¹²⁵I-C2I/AcLDL has been taken up by the lesion. Both Sudan IV staining and autoradiography gave negative results when the aortae of control rabbits were used (data not shown). Figure 2 shows the results obtained with rabbits fed with cholesterol plus anti-atherogenic agent. Lesions in this group were noticeably less severe than those in the non-treated group.

In both cases the radioimages obtained using C2I / AcLDL showed excellent correlation with both the lipid-staining results and the histological examination. Of particular note is the fact that radioimages revealed not only the severely lesioned aortic arches, but also other lightly lesioned areas. Thus, C2I and similar radioimaging probes could be used to detect very early atherosclerotic lesions, which occur at a stage when the disease is essentially reversible.

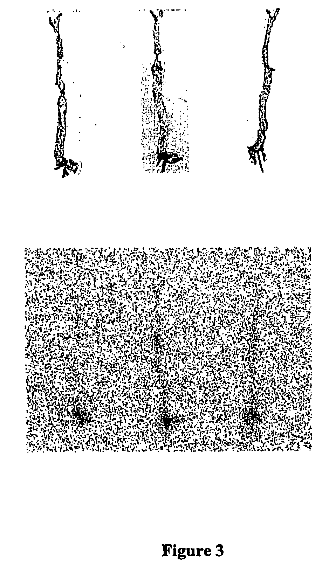

Similar results to those depicted in Figures 1 and 2 were obtained when the double transgenic mice were used (Figure 3), indicating that the use of C2I / AcLDL can be used to selectively image atherosclerotic lesions. A worker skilled in the art would readily appreciate that these result are predictive of the use of C21/AcLDL to selectively image atherosclerotic lesions in humans.

### EXAMPLE 7: Detection of atherosclerotic lesions in a mouse atherosclerosis model using ¹²⁵I-C2I/lipid microemulsions

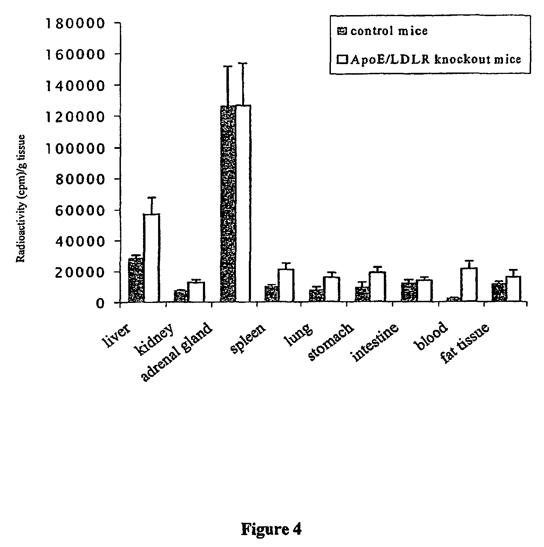

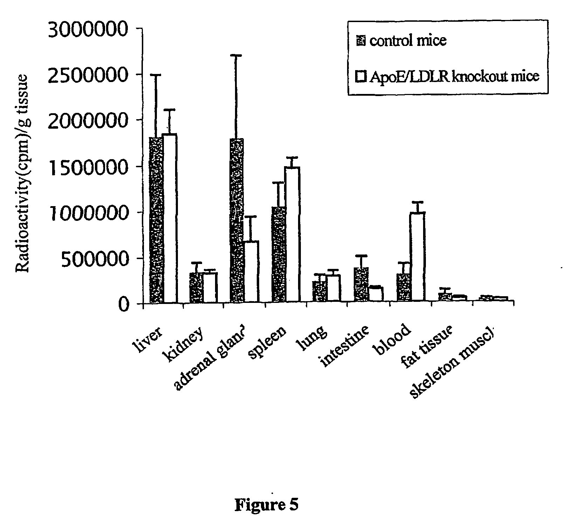

ApoE/LDLR knockout mice (age 7 - 8 weeks, average weight 22g) were maintained on a normal chow diet. The mice were randomly divided into two groups: ¹²⁵I-C2I / AcLDL group (n=6) and ¹²⁵I-C2I / microemulsion group (n=6). C57 mice (age 6 - 7 weeks, n=7) were used as a control group. After 12 weeks, blood samples were taken from the orbital sinus, and plasma cholesterol and triglyceride levels were measured using standard diagnostic kits (Sigma Chemical Company). The ¹²⁵I-C2I / AcLDL preparation (50 Ci/kg) and ¹²⁵I-C21 / microemulsion (80 Ci/kg) were injected into the tail vein of the appropriate group of mice. The mice were sacrificed 24 hours post-injection, and 2% paraformaldehyde fixative was infused into the heart. Different organs were sampled to determine the biodistribution of each ¹²⁵IC2I preparation using a gamma counter. The biodistribution of ¹²⁵I-C2I / AcLDL is shown in Figure 4 and that of ¹²⁵I-C2I / microemulsion is shown in Figure 5. In both cases, accumulation of ¹²⁵I-C2I in the blood was greater in the ApoE/LDLR mice than in the control group.

Based on previous experience, early atherosclerotic lesions were expected to form in the aortas of the ApoE/LDLR knockout mice. Therefore, aortae samples from these mice were removed and washed, then fixed and stained with Sudan IV dye. The aortae samples were subsequently exposed to a Phosphor Screen for 6 - 8 hours.

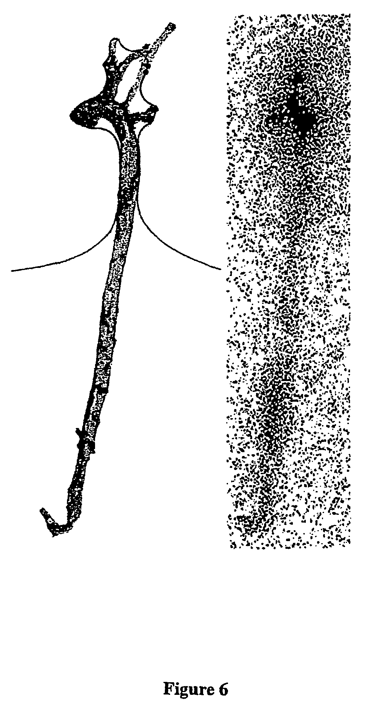

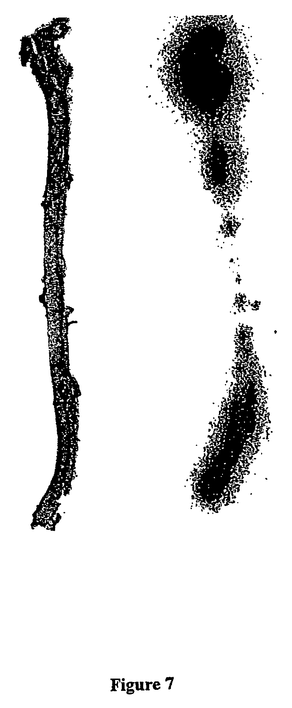

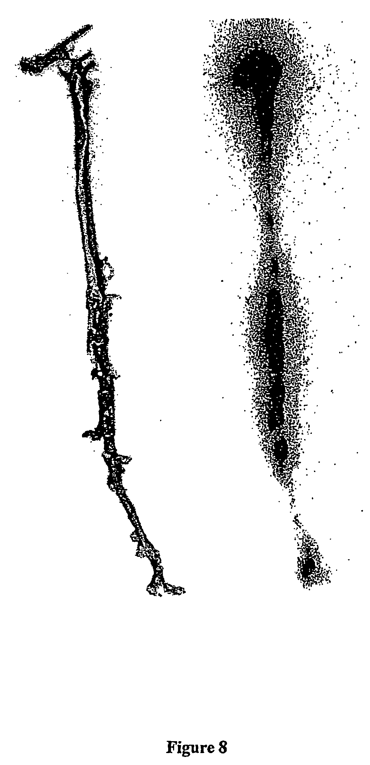

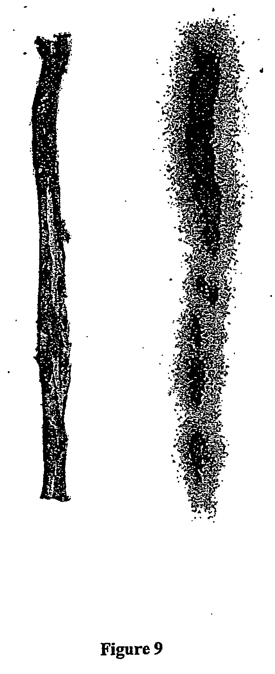

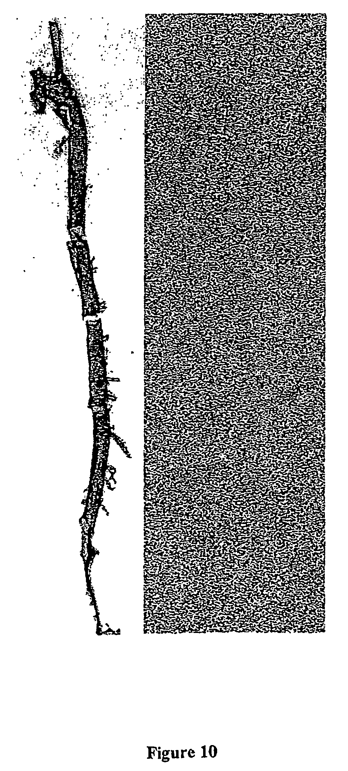

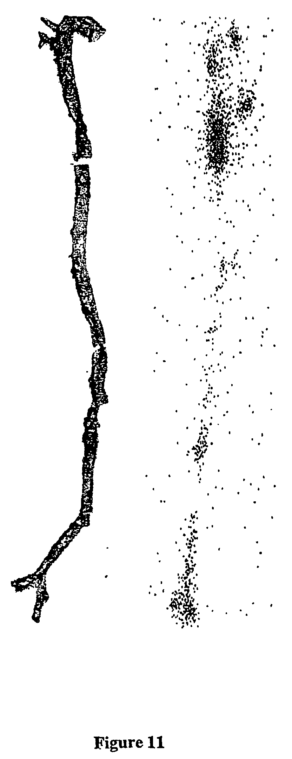

The phosphorimages of aortae of the ApoE/LDLR mice indicated accumulation of ¹²⁵I-C2I / AcLDL (Figures 6 and 7, right panels) or ^{J25}I-C21 / microemulsion (Figures 8 and 9, right panels). Superimposition of the phosphoimages on the Sudan IV stained aortae (Figures 6, 7, 8 and 9, left panels) indicated that ¹²⁵I-C21 accumulation occurred at the lesion sites. Both lipid stain (Figures 10 and 11, left panels) and phosporimage (Figures 10 and 11, right panels) showed negative results with the aortae of the control group of C57 mice. Both ¹²⁵I-C2I / AcLDL and ¹²⁵I-C2I / microemulsion preparations were, therefore, effective in delivering the radiolabeled C2I to the lesion areas.

### EXAMPLE 8: Use of insect lipid transfer protein (iLTP) to load LDL particles

The following agents have been loaded into human LDL using iLTP to provide examples of the utility of this method:
Doxorubicin (Dox), 3'-palmitoyl-[methyl-³H]-2'-deoxythymidine (mpTdR), cholesteryl iopanoate (CI), and 3',5'-dipahnitoyl-5-iodo-deoxyuridine (dp-IUdR). Dox was purchased from Adria Laboratories, Missisauga, ON. CI is a diagnostic imaging agent for early detection of atherosclerosis and was a gift from Dr. Ray E. Counsell of the University of Michigan. Both mp-TdR and dp-IudR were synthesized by the reaction of either [methyl-³H]-2'-deoxythymidine or 5-iodo-2'-deoxyuridine with palmitic acid chloride in dimethylacetamide.

iLTP was isolated and purified according following the previously published protocol [Ryan, *J. Lipid Res.,* **31**:1725-739 (1990); Liu and Ryan, *Biochim. Biophys. Acta,* **1085**:112-118 (1991)].

A typical experiment involved dissolving the drug in an appropriate solvent. After the removal of the solvent under nitrogen, a small quantity of wetting agent was added. An appropriate amount of LDL was then added, based on optimum drug: LDL ratio (for example, 1:1 for Dox) to enhance drug entrapment into LDL. The mixtures were then incubated for 4 hrs at 37 °C in the absence or presence of purified iLTP (typically 20-30 mg of iLTP protein / mg of LDL). After incubation, the mixtures were centrifuged at a density in which the LDL/drug complex floated to the top of the centrifuge tube, while the unbound drug and iLTP remained at the bottom of the tube. The LDL/drug fractions were collected and dialyzed to remove any trace of free drugs remaining. The cholesterol was assayed enzymatically using a commercially available kit (Sigma Chemical Company). The amount of drug associated with LDL was determined by HPLC for Dox, CI, mp-TdR, do-IUdR, by gamma counter for the radiolabeled ¹²⁵I-CI, or by liquid scintillation counting for ³H-mp-TdR. Table 1 and 2 summarize the HPLC conditions and the results of the drug loading into LDL.

**Table 1.**

| ***HPLC conditions using C-18 reverse phase column*** | | | | |
|---|---|---|---|---|
| Drug time | Mobile phase | Flow rate (ml/min.) | Wavelength (nm) | Retention (min.) |
| DOX | 27% CH₃CN in 80 mM acetate buffer (pH 4.0) | 1.0 | 254 | 6.0 |
| | | | | |
| CI | 22% THF in CH₃CN | 1.0 | 254 | 8.5 |
| | | | | |
| mp-TdR | 22% THF in CH₃CN | 1.0 | 254 | 3.4 |
| | | | | |
| Dp-IUdR | 22% THF in CH₃CN | 1.0 | 254 | 6.0 |

**Table 2.**

| ***Effect of LTP on the drug loading into human LDL*** | | |
|---|---|---|
| Drug | No LTP (mole drug/mole LDL) | LTP added (mole drug/mole LDL) |
| DOX | 11.9 | 54 |
| | | |
| CI | 36.7 | 143.4 |
| | | |
| mp-TdR | 85 | 238 |
| | | |
| dp-IudR | 100 | 425 |

Table 2 demonstrates that iLTP enhanced the transfer of drug molecules into LDL by 3-5 fold. In addition, preliminary examination of possible structural and conformational changes after drug loading have been performed with different available techniques and indicated that there were no significant differences between native LDL and drug-loaded LDL (data not shown).

### EXAMPLE 9: Testing receptor-mediated uptake of ¹²⁵I-DPIP/LDL by cervical cancer cell-lines

Cervical cancer cell lines HeLa, SiHa and C-33A were purchased from the ATCC.

### Determination of LDL receptor numbers on HeLa, SiHa and C-33A.

LDL receptor binding was tested following the method described by Goldstein and Brown [*J. Biol. Chem.,* **249**:5153-5162 (1974)] with minor modifications. The HeLa, SiHa and C-33A cell lines were cultured in Dulbecco's Modified Eagle's Medium (DEME) supplemented with 10% heat-inactivated fetal calf serum (FCS), 2 mM essential amino acids, 50 IU penicillin/ml and 50 µg/ml streptomycin at 37°C in a humidified 4% carbon dioxide/air atmosphere in 125 cm² flasks (Costar).

Before study, cells were plated in 34 mm-diameter 8-well culture plates at densities of approximately 3.3x10⁵ (HeLa), 1x10⁶ (SiHa) and 5x10⁵ (C-33A) per well and incubated with LPDS medium (using LPDS to replace the FCS in the medium prepared as indicated above) for 1 day (HeLa, C-33A) or 2 days (SiHa). For the LDL receptor binding experiment, the cells were incubated with various concentrations of ¹²⁵I-LDL in the absence or in the presence of 20-fold excess unlabeled LDL at 4°C. After 4 hours incubation (8 hours for SiHa), the medium was removed and cells in each well were washed three times with 1 mL ice-cold Tris buffer (150 mM NaCl, 50mM Tris, 2mg/mL. BSA; pH 7.4), then three times with 1mL ice-cold PBS buffer. After washing, 1 mL 0.1N NaOH was added to each well to dissolve the cells. A sample of 500 µL was removed from each well and the amount of bound ¹²⁵I-LDL determined by measuring the radioactivity by gamma-counter and by determining the protein concentration by the Bradford method.

The experiments conducted in the presence of an excess amount of unlabeled LDL allowed the non-specific binding of ¹²⁵I-LDL to the cells to be measured. The specific receptor mediated binding of ¹²⁵I-LDL could then be determined by subtracting the non-specific binding from the total binding. The LDL receptor number (Bₘₐₓ) and dissociation constant (K_{d}) for each cell line were determined by GraphPad Prism™ software. The results are shown in Table 3 below.

**Table 3.**

| ***LDL receptor number (B***_{***max***}***)***^{***a***} ***of, and the dissociation constant (K***_{***d***}***)***^{***b***} ***of the*** ***specific binding of LDL to HeLa, SiHa, and C-33A cells*** | | | |
|---|---|---|---|
| | HeLa | SiHa | C-33A |
| Bₘₐₓ | 38 314±9 873 | 70 304±6 180 | 120 355±21 781 |
| | | | |
| K_{d} | 33.98±16.03 | 23.23±4.42 | 49.85±20.18 |

| | | | |
|---|---|---|---|
| ^{a} Expressed as LDL receptor number per cell ± SEM; n=4. | | | |
| ^{b} Expressed as nM ± SEM; n=4. | | | |

HeLa is the most commonly used cervical tumour cell line for cytotoxic studies of anticancer drug/LDL complexes. These results indicated that this cell line has a high expression of LDL receptor (38 314 ± 9 873 per cell) consistent with the figure of 40,000 receptors per cell obtained in previous studies [Lestavel-Delattre, *et al., Cancer Res.,* **52**:3629-3635 (1992)]. These results further indicated that the LDL receptor numbers of two other cervical cancer cell lines, SiHa and C-33A, were twice (about 70,000 receptors per cell) and three times (about 120,000 receptors per cell) respectively those found for the HeLa cell line. Thus indicating that high numbers of LDL receptors is a common feature of cervical cancer cells.

### Test of receptor-mediated uptake of ¹²⁵I DPIP/LDL by cells.

Before study, cells were grown as described above for the LDL binding experiment. During the receptor-mediated uptake experiment, the cells were incubated at 37°C for 4 hours with various concentrations of either ¹²⁵I-DPIP / LDL, ¹²⁵I-DPIP / LDL plus 20-fold excess natural LDL, or ¹²⁵I-DPIP / LDL plus 20-fold excess AcLDL. After incubation, the medium was removed and cells in each well were washed three times with 1 mL ice-cold Tris buffer (150 mM NaCl, 50mM Tris, 2mg/mL BSA; pH 7.4), then three times with 1mL ice-cold PBS buffer. After washing, 1 mL 0.1N NaOH was added to each well to dissolve the cells. A sample of 500 µL was removed from each well and the amount of bound ¹²⁵I-LDL determined by measuring the radioactivity by gamma-counter and by determining the protein concentration by the Bradford method.

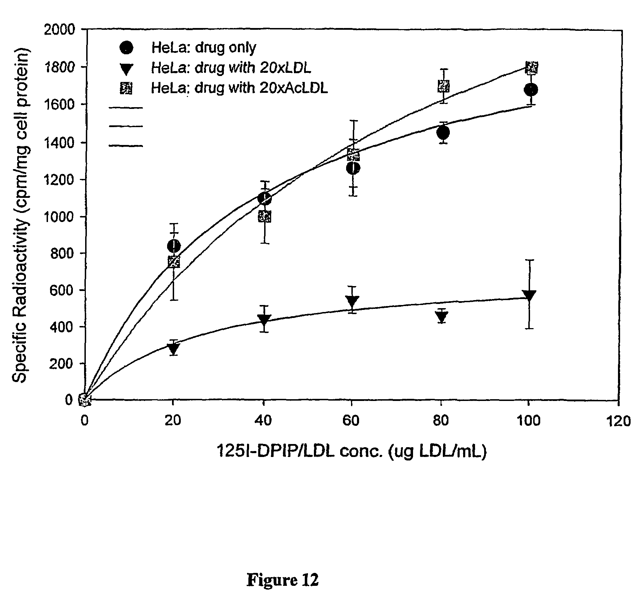

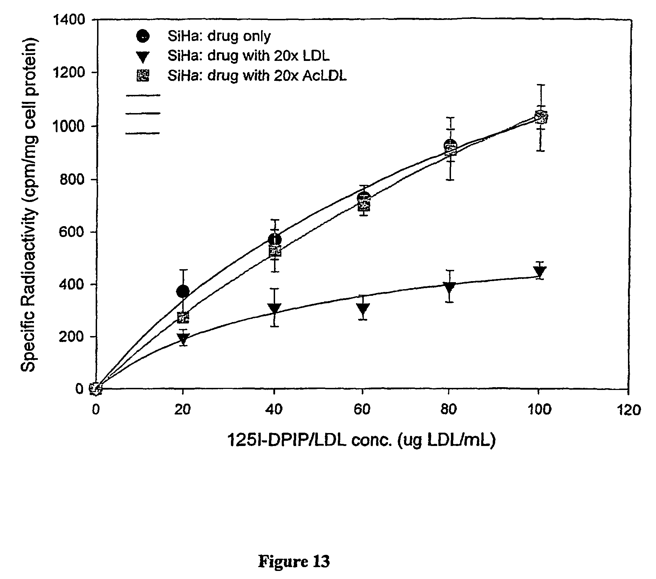

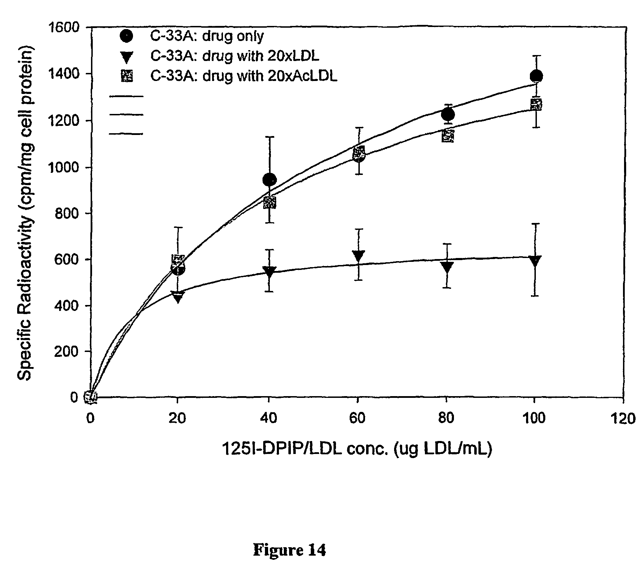

In these competitive uptake studies, uptake of ¹²⁵I-DPEP/LDL was seen to be significantly lowered in all three cell lines when cells were co-incubated with 20-fold excess native LDL (p<0.05 in all three cell studies, see Figures 12, 13 and 14). This result indicates that the drug / LDL complex competes with native LDL for the LDL receptors on each cell which is consistent with these results (p>0.05 in all three cell studies). These results also indicated that AcLDL does not compete significantly with the ¹²⁵I-DPIP / LDL complex for the LDL receptors (p>0.05 in all three cell studies, see Figures 12, 13 and 14), which is consistent with previous reports that AcLDL is not identified by or bound by LDL receptors [Goldstein *et al.,Proc. Natl. Acad. Sci. USA,* **76**:333-337 (1979)]. These experiments indicate that the ¹²⁵I-DPIP / LDL complex will be specifically internalized by tumour cells through an LDL receptor-mediated pathway, thus indicating the utility of ¹²⁵I-DPIP/LDL and related compounds as tumour imaging agents.

### EXAMPLE 10: Detection of atherosclerotic lesions in a miniature swine atherosclerosis model using ¹²⁵I-C2I/AcLDL or ¹²⁵I-C2I/microemulsion

Miniature swine are maintained on a egg yolk (11%) / cholesterol (1%) enriched atherogenic diet for 4-6 months [Rolland, *et al., Am. J. Cardiol.,* **71**:E22-E27 (1993); Prescott, *et al., Atherosclerosis X,* 101-104 (1994)]. Iodine is supplemented in the diets. Under these conditions, the animals develop lesions that resemble human atherosclerotic plaques. ¹²⁵I-C2I / AcLDL or ¹²⁵I-C2I /microemulsion is injected into the pigs intravenously. At different time intervals blood samples are collected. The animals are terminated at appropriate times and all major organs will be collected and weighed. Radioimaging is carried out as described above for the rabbit and transgenic mouse models with changes necessitated by the difference in the size of the animals.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A compound of the formula (I): 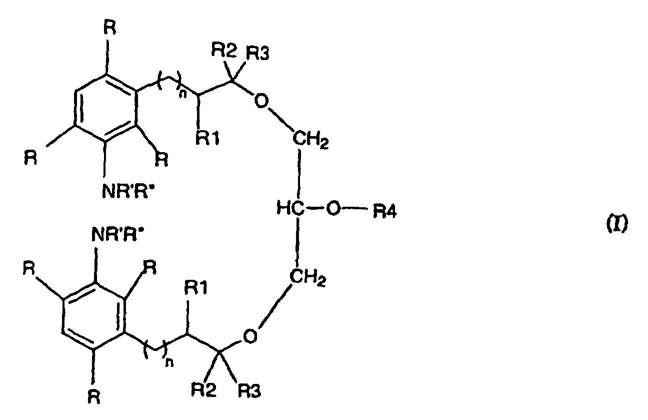 wherein:
n is an integer betweenland 16;
each R group is independently H or I, wherein between 2 to 6 R groups must I;
R' and R" are independently H, (C1-C4 alkyl) or an amine protecting group;
R1 is H or (C₁-C₆) alkyl;
R2 is H;
R3 is H or when taken together R2 and R3 is =O; and
R4 is: 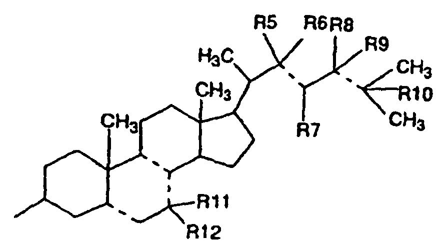 wherein:
the bond - - - designates a single or a double bond;
R5 is H, OR"' or R"', wherein R"' is (C₁-C₆) alkyl;
R6 is H or absent;
R7 is H or absent;
R8 is H, (C₁-C₆) alkyl or (C₂-C₆) alkenyl;
R9 is H or absent;
R10 is H or absent;
R11 and R12 are independently H, R"' or OR"', or when taken together R11 and R12 are =O;
with the proviso that when R9 and R10 are absent and the bond - - - between carbon atoms bearing R9 and R10 is a double bond, then R8 is H or (C₁-C₆) alkyl; and that two adjacent bonds designated as - - - are not double bond at the same time.

2. The compound according to Claim 1 wherein R4 is β-sitosterol, dihydrocholesterol, 7-dehydrocholesterol, 22-hydroxycholesterol, 25-hydroxycholesterol, 3-cholesten-3β-of-7-one, 5α-cholestane-3β-ol, 5α-cholest-7-en-3β-ol, 7β-hydroxycholesterol, campesterol, desmosterol, ergosterol, fucosterol, lanosterol and stigmasterol.

3. The compound according to Claim 1 wherein R4 is cholesterol.

4. The compound according to Claim 1 wherein n = 1, R1 is C₂H₅, and R2 and R3 together are =O.

5. A compound of the structure:

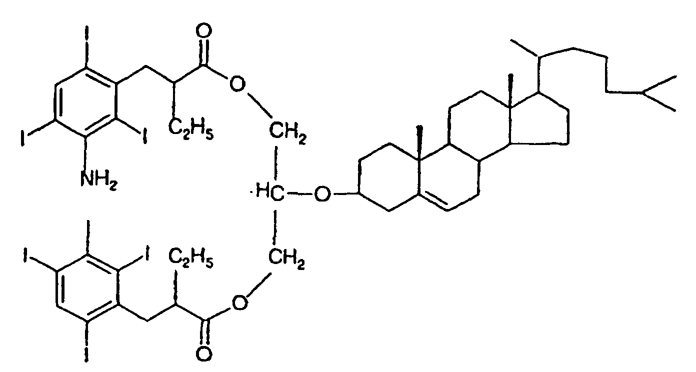

6. The compound according to any one of Claims 1, 2, 3 or 5 wherein I is selected from the group consisting of ¹²³I, ¹²⁵I and ¹³¹I.

7. A composition comprising the compound according to Claim 6 and a delivery vehicle.

8. The composition according to Claim 7 wherein said delivery vehicle is a low density lipoprotein (LDL).

9. The composition according to Claim 8 wherein the LDL is a modified LDL.

10. The composition according to Claim 9 wherein the modified LDL is acetylated LDL.

11. The composition according to Claim 7 wherein said delivery vehicle is a synthetic microemulsion or a liposome.

12. A compound according to Claim 6, for use in a method of diagnostic radioimaging.

13. A compound according to Claim 12, for use in visualising atherosclerotic lesions, liver cells, neural, cells or tumour cells.

14. A compound according to Claim 12, for use in a method of diagnosing atherosclerosis, stroke, cirrhosis, hepatitis, Alzheimer disease, glomerulosclerosis, ataxia with Vitamin E deficiency, liver tumours, or cancer.

15. A compound according to Claim 12, for use in detection of hepatic disease or for detection and localization of tumours in a mammal by radioimaging.

16. A composition according to any one of Claims 7 to 11, for use in a method of diagnostic radioimaging.

17. A composition according to Claim 16, for use in visualising atherosclerotic lesions, liver cells, neural cells or tumour cells.

18. A composition according to Claim 16, for use in a method of diagnosing atherosclerosis, stroke, cirrhosis, hepatitis, Alzheimer disease, glomerulosclerosis, ataxia with Vitamin E deficiency, liver tumours, or cancer.

19. A composition according to Claim 16, for use in detection of hepatic disease or for detection and localization of tumours in a mammal by radioimaging.

20. Use of a compound according to any one of claims 1 to 5 for the manufacture of a radioimaging agent.

21. A diagnostic kit comprising the compound according to any one of Claims 1 - 5, a suitable delivery vehicle, reagents necessary for the radiolabelling of the compound with an appropriate radioisotope of iodine and instructions for use.

22. A kit for preparing radiolabeled probes comprising the compound of any one of Claims 1 - 5, reagents necessary for the radiolabelling of the compound with an appropriate radioisotope of iodine and instructions for use.

23. A kit for preparing a radiolabeled diagnostic composition for use in radioimaging, comprising the compound according to any one of Claims 1 - 5, emulsifying agents and core lipids necessary to prepare a microemulsion, reagents necessary for the radiolabelling of the compound with an appropriate radioisotope of iodine and instructions for use.

## Patentansprüche

1. Verbindung der Formel (I): 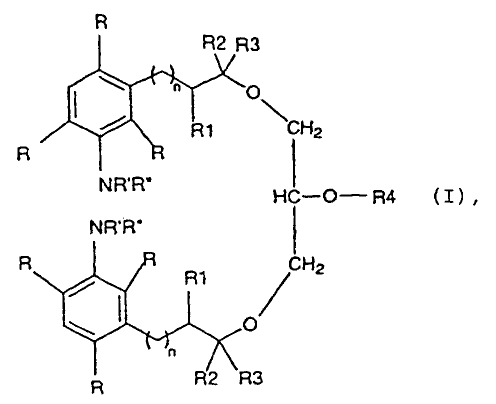 wobei:
n eine Zahl zwischen 1 und 16 ist,
jede R-Gruppe unabhängig H oder I ist, wobei zwischen 2 und 6 R-Gruppen I sein müssen,
R' und R" unabhängig H, (C₁-C₄) -Alkyl oder eine Aminschutzgruppe sind,
R1 H oder (C₁-C₆)-Alkyl ist,
R2 H ist,
R3 H ist, oder R2 und R3 zusammengefaßt =O bedeuten, und
R4 ist: 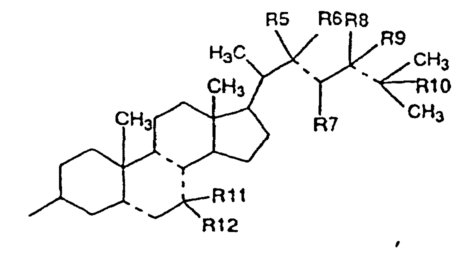 wobei:
die Bindung --- eine Einfach- oder Doppelbindung bedeutet,
R5 H, OR' ' ' oder R' ' ' ist, wobei R' ' ' (C₁-C₆)-Alkyl ist,
R6 H ist oder fehlt,
R7 H ist oder fehlt,
R8 H, (C₁-C₆) -Alkyl oder (C₂-C₆) -Alkenyl ist,
R9 H ist oder fehlt,
R10 H ist oder fehlt,
R11 und R12 unabhängig H, R' ' ' oder OR' ' ' sind, oder R11 und R12 zusammengefaßt =O sind,
mit der Maßgabe, daß, wenn R9 und R10 fehlen und die Bindung --- zwischen den R9 und R10 tragenden Kohlenstoffatomen eine Doppelbindung ist, R8 dann H oder (C₁-C₆)-Alkyl ist, und daß zwei benachbarte Bindungen, die als --- bezeichnet sind, nicht gleichzeitig Doppelbindungen sind.

2. Verbindung gemäß Anspruch 1, wobei R4 β-Sitosterol, Dihydrocholesterin, 7-Dehydrocholesterin, 22-Hydroxycholesterin, 25-Hydroxycholesterin, 3-Cholesten-3β-ol-7-on, 5α-Cholestan-3β-ol, 5α-Cholest-7-en-3β-ol, 7β-Hydroxycholesterin, Campesterol, Desmosterol, Ergosterol, Fucosterol, Lanosterol und Stigmasterol ist.

3. Verbindung gemäß Anspruch 1, wobei R4 Cholesterin ist.

4. Verbindung gemäß Anspruch 1, wobei n = 1, R1 C₂H₅ ist und R2 und R3 zusammen =O sind.

5. Verbindung der Struktur:

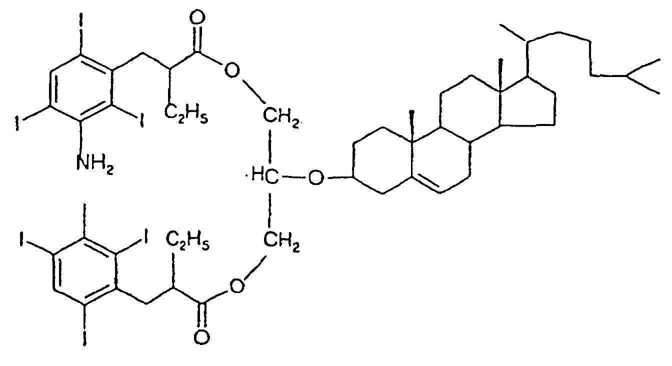

6. Verbindung gemäß irgendeinem der Ansprüche 1, 2, 3 oder 5, wobei I ausgewählt ist aus der Gruppe, bestehend aus ¹²³I, ¹²⁵I und ¹³¹I.

7. Zusammensetzung, welche die Verbindung gemäß Anspruch 6 und ein Trägervehikel umfaßt.

8. Zusammensetzung gemäß Anspruch 7, wobei das genannte Trägervehikel ein Low-Density-Lipoprotein (LDL) ist.

9. Zusammensetzung gemäß Anspruch 8, wobei das LDL ein modifiziertes LDL ist.

10. Zusammensetzung gemäß Anspruch 9, wobei das modifizierte LDL acetyliertes LDL ist.

11. Zusammensetzung gemäß Anspruch 7, wobei das genannte Trägervehikel eine synthetische Mikroemulsion oder ein Liposom ist.

12. Verbindung gemäß Anspruch 6 zur Verwendung bei einem Radioimaging-Diagnoseverfahren.

13. Verbindung gemäß Anspruch 12 zur Verwendung bei der Sichtbarmachung von atherosklerotischen Läsionen, Leberzellen, Nervenzellen oder Tumorzellen.

14. Verbindung gemäß Anspruch 12 zur Verwendung bei einem Verfahren zur Diagnose von Atherosklerose, Apoplexie, Zirrhose, Hepatitis, Alzheimer-Krankheit, Glomerulosklerose, Ataxie mit Vitamin-E-Mangel, Lebertumoren oder Krebs.

15. Verbindung gemäß Anspruch 12 zur Verwendung bei der Erkennung einer Lebererkrankung oder zur Erkennung und Lokalisierung von Tumoren bei einem Säugetier durch Radioimaging.

16. Zusammensetzung gemäß irgendeinem der Ansprüche 7 bis 11 zur Verwendung bei einem Radioimaging-Diagnoseverfahren.

17. Zusammensetzung gemäß Anspruch 16 zur Verwendung bei der Sichtbarmachung von atherosklerotischen Läsionen, Leberzellen, Nervenzellen oder Tumorzellen.

18. Zusammensetzung gemäß Anspruch 16 zur Verwendung bei einem Verfahren zur Diagnose von Atherosklerose, Apoplexie, Zirrhose, Hepatitis, Alzheimer-Krankheit., Glomerulosklerose, Ataxie mit Vitamin-E-Mangel, Lebertumoren oder Krebs.

19. Zusammensetzung gemäß Anspruch 16 zur Verwendung bei der Erkennung einer Lebererkrankung oder zur Erkennung und Lokalisierung von Tumoren bei einem Säugetier durch Radioimaging.

20. Verwendung einer Verbindung gemäß irgendeinem der Ansprüche 1 bis 5 zur Herstellung eines Radioimaging-Mittels.

21. Diagnosekit, umfassend die Verbindung gemäß irgendeinem der Ansprüche 1-5, ein geeignetes Trägervehikel, Reagenzien, die zur Radiomarkierung der Verbindung mit einem geeigneten Radioisotop von Iod notwendig sind, und eine Gebrauchsanleitung.

22. Kit zur Herstellung radiomarkierter Proben, umfassend die Verbindung nach irgendeinem der Ansprüche 1-5, Reagenzien; die zur Radiomarkierung der Verbindung mit einem geeigneten Radioisotop von Iod notwendig sind, und eine Gebrauchsanleitung.

23. Kit zur Herstellung einer radiomarkierten Diagnosezusammensetzung zur Verwendung beim Radioimaging, umfassend die Verbindung gemäß irgendeinem der Ansprüche 1-5, Emulgatoren und Kernlipide, die zur Herstellung einer Mikroemulsion notwendig sind, Reagenzien, die zur Radiomarkierung der Verbindung mit einem geeigneten Radioisotop von Iod notwendig sind, und eine Gebrauchsanleitung.

## Revendications

1. Un composé de formule (I) : 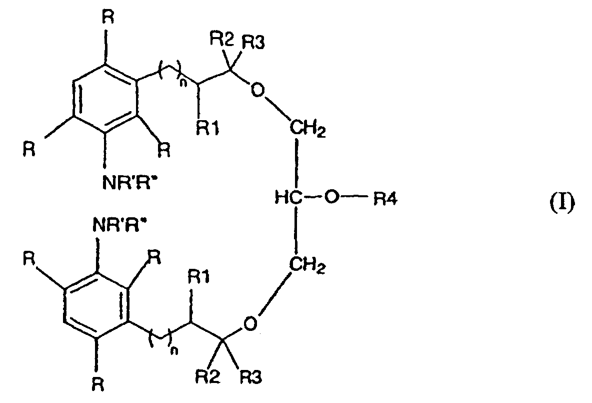 dans laquelle :
n est un entier entre 1 et 16 ;
chaque groupement R est indépendamment H ou I, dans laquelle entre 2 à 6 groupements R doivent être I ;
R' et R" sont indépendamment H, un alkyle en C₁ à C₄ ou un groupement protecteur d'amine ;
R₁ est H ou un alkyle en C₁ à C₆ ;
R₂ est H ;
R₃ est H ou, lorsqu'ils sont pris ensemble, R₂ et R₃ sont =O et
R₄ est : 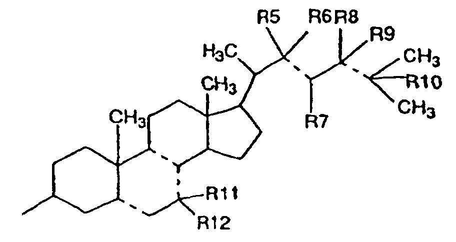 dans laquelle :
la liaison - - - désigne une liaison simple ou double ;
R₅ est H, OR"' ou R"', dans laquelle R"' est un alkyle en C₁ à C₆ ;
R₆ est H ou absent ;
R₇ est H ou absent ;
R₈ est H, un alkyle en C₁ à C₆ ou un alcényle en C₂ à C₆ ;
R₉ est H ou absent ;
R₁₀ est H ou absent ;
R₁₁ et R₁₂ sont indépendamment H, R"' ou OR"' ou, lorsqu'ils sont pris ensemble, R₁₁ et R₁₂ sont =O ;
avec la condition que lorsque R₉ et R₁₀ sont absents et la liaison - - - entre les atomes de carbone portant R₉ et R₁₀ est une liaison double, R₈ est alors H ou un alkyle en C₁ à C₆ ;
et que deux liaisons adjacentes, désignées comme - - -, ne sont pas une liaison double en même temps.

2. Le composé selon la revendication 1, dans lequel R₄ est du β-sitostérol, du dihydrocholestérol, du 7-déhydrocholestérol, du 22-hydroxycholestérol, du 25-hydroxycholestérol, du 3-cholesten-3β-ol-7-one, du 5α-cholestane-3β-ol, du 5α-cholest-7-en-3β-ol, du 7β-hydroxycholestérol, du campestérol, du desmostérol, de l'ergostérol, du fucostérol, du lanostérol et du stigmastérol.

3. Le composé selon la revendication 1, dans lequel R₄ est du cholestérol.

4. Le composé selon la revendication 1, dans lequel n = 1, R₁ est C₂H₅, et R₂ et R₃ sont ensemble =O.

5. Un composé de structure :

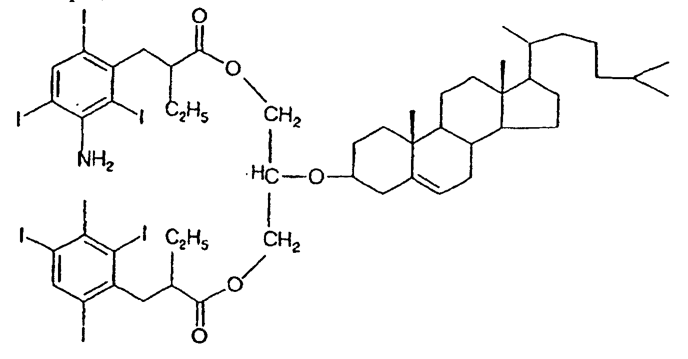

6. Le composé selon l'une quelconque des revendications 1, 2, 3 ou 5, dans lequel I est choisi parmi le groupe constitué de ¹²³I, ¹²⁵I et ¹³¹I.

7. Une composition comprenant le composé selon la revendication 6 et un vecteur.

8. La composition selon la revendication 7, dans laquelle ledit vecteur est une lipoprotéine de basse densité (BLP).

9. La composition selon la revendication 8, dans laquelle la BLP est une BLP modifiée.

10. La composition selon la revendication 9, dans laquelle la BLP modifiée est de la BLP acétylée.

11. La composition selon la revendication 7, dans laquelle ledit vecteur est une micro-émulsion synthétique ou un liposome.

12. Un composé selon la revendication 6, pour une utilisation dans un procédé de diagnostic par radio-imagerie.

13. Un composé selon la revendication 12, pour une utilisation dans la visualisation de lésions athéroscléreuses, de cellules hépatiques, de cellules nerveuses ou de cellules tumorales.

14. Un composé selon la revendication 12, pour une utilisation dans un procédé de diagnostic de l'athérosclérose, des accidents vasculaires, de la cirrhose, de l'hépatite, de la maladie d'Alzheimer, de la glomérulosclérose, d'une ataxie avec déficience en vitamine E, des tumeurs du foie, ou du cancer.

15. Un composé selon la revendication 12, pour une utilisation dans la détection de maladies du foie ou pour la détection et localisation par radio-imagerie de tumeurs chez un mammifère.

16. Une composition selon l'une quelconque des revendications 7 à 11, pour une utilisation dans un procédé de diagnostic par radio-imagerie.

17. Une composition selon la revendication 16, pour une utilisation dans la visualisation de lésions athéroscléreuses, de cellules hépatiques, de cellules nerveuses ou de cellules tumorales.

18. Une composition selon la revendication 16, pour une utilisation dans un procédé de diagnostic de l'athérosclérose, des accidents vasculaires, de la cirrhose, de l'hépatite, de la maladie d'Alzheimer, de la glomérulosclérose, d'une ataxie avec déficience en vitamine E, des tumeurs du foie, ou du cancer.

19. Une composition selon la revendication 16, pour une utilisation dans la détection de maladies du foie ou pour la détection et localisation par radio-imagerie de tumeurs chez un mammifère.

20. Utilisation d'un composé selon l'une quelconque des revendications 1 à 5, pour la fabrication d'un agent de radio-imagerie.

21. Un kit de diagnostic comprenant le composé selon l'une quelconque des revendications 1 à 5, un vecteur approprié, des réactifs nécessaires pour le radio-marquage du composé avec un radio-isotope approprié de l'iode, et des instructions pour l'utilisation.

22. Un kit pour préparer des sondes radio-marquées comprenant le composé selon l'une quelconque des revendications 1 à 5, des réactifs nécessaires pour le radio-marquage du composé avec un radio-isotope approprié de l'iode, et des instructions pour l'utilisation.

23. Un kit pour préparer une composition radio-marquée de diagnostic pour une utilisation en radio-imagerie, comprenant le composé selon l'une quelconque des revendications 1 à 5, des agents émulsifiant et des nucléolipides nécessaires pour préparer une micro-émulsion, des réactifs nécessaires pour le radio-marquage du composé avec un radio-isotope approprié de l'iode, et des instructions pour l'utilisation.
